# EUROPEAN PATENT APPLICATION

(11) **EP 1 484 069 A1**
(43) Date of publication of application: **08.12.2004**
(21) Application number: 02767861.4
(22) Date of filing: 27.08.2002
(51) Int. Cl.: A61L 2/06, A61B 1/00

(54) **STERILIZATION APPARATUS AND CONTAINER FOR STERILIZATION**

(30) Priority: 28.02.2002 JP 2002054819
(71) Applicant: Olympus Corporation, Tokyo 151-0072 (JP)
(72) Inventor: HASEGAWA, Hitoshi, Yokohama-shi, Kanagawa 222-0002 (JP); SUZUKI, Eiri, Sagamihara-shi, Kanagawa 229-0038 (JP); KUROSHIMA, Hisashi, Hachioji-shi, Tokyo 193-0832 (JP); NOGUCHI, Toshiaki, Tachikawa-shi, Tokyo 190-0002 (JP); MORIYAMA, Hiroki, Akishima-shi, Tokyo 196-0032 (JP)
(74) Representative: von Hellfeld, Axel, Dr. Dipl.-Phys.
(86) International application number: PCT/JP2002/008623
(87) International publication number: WO 2003/072146

(57) **Abstract**

A sterilization apparatus is constituted of a chamber (4) in which a material to be sterilized is stored to sterilize the material by steam having a high temperature and pressure, and a cooling mechanism (30, 31) which cools the material in a thermally isolated state from the inside of the chamber. Accordingly, there can be provided a sterilization apparatus in which the material to be sterilized can rapidly be cooled without troubling an operator or without cooling the inside of the chamber and which realizes load reduction of a sterilization operation and reduction of a time in a cycle.

## Description

### Technical Field

The present invention relates to a sterilization apparatus and a container for sterilization.

### Background Art

First, a first related art will be described. In Jpn. Pat. Appln. KOKAI Publication No. 2000-202006, a sterilization apparatus is disclosed in which a mechanism for automatically partly opening/closing a door of the unit is disposed to discharge steam for sterilization via the partly open door in a drying step. Moreover, in Japanese Utility Model Application Laid-Open No. 7-14264, the door is constituted to be movable up and down and rotatable by a support arm disposed on a pressure container main body, and to be automatically openable/closable by expansion/contraction of a power cylinder disposed on a side surface of the pressure container main body.

Moreover, to quickly use materials to be sterilized such as an endoscope in the conventional sterilization apparatus, the materials to be sterilized have been removed from a sterilization case or pack and exposed to a cold blast or water, or the cold blast has been applied to the outside of the sterilization case or pack to cool the materials.

Next, a second related art will be described. To subject the endoscope, which is a precision electronic instrument, to high pressure steam sterilization (autoclave) means that the endoscope is exposed to very harsh conditions. Therefore, as compared with an endoscope on the premise that the endoscope be used in general disinfection/sterilization means, various countermeasures such as a high pressure countermeasure, steam countermeasure, and a high temperature countermeasure have been taken.

In an autoclave device, after the endoscope is usually sterilized with high pressure steam at about 130°C, a drying step is performed, and the steam stuck to the endoscope is dried. During this, to evaporate and dry the steam in a short time, while the inside of a chamber is kept at the high temperature, a plurality of vacuum drawing steps are further performed.

A reason why the chamber is not positively cooled is that the next sterilization treatment be performed efficiently (in the short time). In a general autoclave device, after the drying step ends, the material to be sterilized is removed from the chamber and left to stand. Therefore, for a cooling method, natural cooling is the mainstream.

In Jpn. Pat. Appln. KOKAI Publication No. 6-142162, the following apparatus is proposed in order to efficiently cool the material to be sterilized. The proposed apparatus includes: a base; a steam generation unit including an opening above the base and an electric heating material in an inner bottom portion capable of receiving water; a cylindrical sterilization chamber container which is attached to the opening of the steam generation unit via air-tight means and which includes an opening; a steam discharge conduit connected to the steam generation unit via a steam valve; and a lock mechanism for locking and unlocking the sterilization chamber container with respect to the steam generation unit.

By this constitution, the attaching/detaching of the sterilization chamber container is possible, insertion/removal of instruments to be sterilized is facilitated, and steam is dissipated after the sterilization ends. The sterilization chamber container is quickly unlocked and removed, and the instrument to be sterilized can quickly be cooled. Furthermore, a technique of driving a cooling fan after the unlocking to promote the cooling is also disclosed.

Next, a third related art will be described.

An endoscope for medical application has heretofore broadly been used in which an elongated insertion portion is inserted into a body cavity to observe an organ in the body cavity, or a treatment instrument passed through a treatment instrument channel can be used to perform various medical treatments.

Especially, for an endoscope for use in a medical field, the insertion portion is inserted in the body cavity to observe the organs, or the treatment instrument inserted in the treatment instrument channel of the endoscope is used to perform various cures and treatments.

Therefore, when the endoscope or the treatment instrument once used is reused for another patient, infection between the patients via the endoscope or the treatment instrument needs to be prevented, and therefore an endoscope apparatus has to be cleaned/disinfected after inspection/treatment ends.

In recent years, autoclave sterilization (high pressure steam sterilization) which does not involve any intricate operation and which can be used immediately after sterilized and whose running cost is small has been the mainstream of the disinfection/sterilization treatment of the endoscope apparatus.

For example, in Jpn. Pat. Appln. KOKAI Publication No. 5-337081, a case for endoscope sterilization is disclosed in which the high pressure steam sterilization is possible in a cased state of the endoscope. Moreover, while a sterile state is held, movement/storage is possible.

A problem of the above-described first related art will be described. When the number of medical instruments is limited, the sterilized material is sometimes used in the next inspection or treatment immediately after the sterilization. In this case, an operator needs to quickly remove the material to be sterilized from the chamber and cool the material. This has been a troublesome operation.

Moreover, when the door is constituted to be automatically openable in order to reduce the operation time, the temperature in the chamber drops, a heat insulation effect in the next sterilization step is eliminated, and therefore a disadvantage that the steam for the sterilization concentrates sometimes occurs. Therefore, there has been a demand for a sterilization apparatus which can efficiently and automatically cool only the material to be sterilized.

Furthermore, in the method of removing the material to be sterilized from the sterilization case or pack to apply the cold blast or water to the material, care needs to be taken in handling the material so as to prevent the sterilized material from being contaminated. Moreover, the method of applying the cold blast to the outside of the sterilization case or pack to cool the material has a disadvantage that cooling efficiency is low.

The problem of the above-described second related art will be described. In general, the endoscope inspection is performed with respect to 5 to 20 cases in one day per institution. To efficiently perform the inspection, it is necessary to efficiently perform the cleaning/disinfecting treatment or the cleaning/sterilizing treatment of the endoscope in the time between the cases. In the present situation, manual preliminary cleaning and actual cleaning/disinfecting by an automatic endoscope cleaning/disinfecting machine are performed, and this is performed by the following process.

First, when the endoscope inspection ends, the preliminary cleaning (cleaning of an outer surface of the endoscope, air/water feed in the channel) is performed at bed side, and next the endoscope is set on the automatic endoscope cleaning/disinfecting machine. The automatic endoscope cleaning/disinfecting machine has a function of cleaning and rinsing the outer surface of the scope and the inside of the channel, and also has a function of thereafter disinfecting the endoscope with an antiseptic solution and again rinsing the endoscope. Furthermore, there is also a product which has a function of drying the inside of the channel.

Nowadays, there has also been a need for the sterilization of the endoscope because of appearance of new pathogens, in the present situation, the sterilization treatment by an EOG gas has been performed, but there are problems that a sterilization treatment time of the EOG gas is long and ease of handling has to be solved. Therefore, the high pressure steam sterilization (autoclave) which has no such problems has been used.

A large number of components are mounted inside the endoscope, and it has been assumed to be difficult to sterilize the endoscope in the autoclave, but in recent years, heat resistance of the components or adhesive has also been enhanced. The sterilization has been possible even on conditions of the autoclave such as a condition at 135°C for five minutes.

When the endoscope is sterilized in the general autoclave device described in the related art, the endoscope is treated in a usual step, and the sterilizing to the drying can be ended. However, at this time, an endoscope main body is held at a temperature of about 70°C. Even when the main body is removed from the device and naturally cooled, a time of about 30 minutes is required to obtain a temperature at which the endoscope is usable.

Furthermore, for the autoclave device, sordes stuck during operation need to be cleaned before the treatment. When the steps of the preliminary cleaning and real cleaning are also included before the treatment in the autoclave device, an enormous time is required in a cleaning/sterilizing treatment between the cases in the above-described state of natural cooling, and there is a problem that the inspection cannot efficiently be performed. That is, after ending one case, much time is required before starting the next case, the number of inspectable times in one day sharply decreases, and therefore the method is also economically disadvantageous.

The problem of the above-described third related art will be described.

In the case for the endoscope sterilization in the Jpn. Pat. Appln. KOKAI Publication No. 5-337081, a means for the cooling in a short time has not been provided such that the endoscope can be used in the inspection immediately after performing the high pressure steam sterilization. Therefore, there has been a disadvantage that there is a standby state until the endoscope is cooled at a temperature of 40°C or less at which the endoscope can be inserted in the interior of the body.

Therefore, a first object of the present invention is to provide a sterilization apparatus in which a material to be sterilized can rapidly be cooled without any operator's trouble or without cooling the inside of the chamber so as to realize reduction of a load of a sterilization operation and reduction of a time in a cycle.

A second object of the present invention is to provide a sterilization apparatus in which the material to be sterilized is efficiently cooled and is immediately brought in a reusable state so as to promote efficiency of in-hospital services.

A third object of the present invention is to provide a sterilization apparatus in which a cooling time of an endoscope after high pressure steam sterilization is reduced and user's usability is enhanced and efficient endoscope instrument can be performed.

A fourth object of the present invention is to provide a container for sterilization in which the endoscope is quickly cooled without being contaminated immediately after autoclave sterilization.

According to a first aspect of the present invention, there is provided a sterilization apparatus comprising:
a chamber in which a material to be sterilized is stored to sterilize this material by steam having a high temperature and pressure; and
a cooling mechanism which cools the material in a thermally isolated state from the inside of the chamber.

Moreover, according to a second aspect of the present invention, there is provided a container for sterilization comprising:
a containing section in which a material to be sterilized is stored;
an intake port and an exhaust port of a gas, at least either one of which is connectable to a gas circulation mechanism to circulate the gas inside the containing section; and
a germ trapping filter disposed in at least the intake port.

Furthermore, according to a third aspect of the present invention, there is provided a container for sterilization comprising:
a containing section in which a material to be sterilized is stored;
a connection section via which a fluid to sterilize and cool the stored material to be sterilized is supplied/discharged with respect to the outside; and
a blocking mechanism which is disposed in the connection section and which can communicate to supply/discharge the fluid in a connected state of the connection section and which blocks off in a non-connected state.

Additionally, according to a fourth aspect of the present invention, there is provided a sterilization apparatus, comprising:
a container for sterilization including a containing section in which a material to be sterilized is stored, a connection section which supplies/discharges a fluid to sterilize/cool the stored material to be sterilized with respect to the outside, and a blocking mechanism which is disposed in the connection section and which can communicate to supply/discharge the fluid in a connected state of the connection section and which blocks off in a non-connected state;
a chamber in which the material to be sterilized or the container for sterilization is stored to sterilize the material by steam having a high pressure and temperature; and
fluid supply means capable of supplying at least one of a sterilization fluid to sterilize the material to be sterilized and a cooling fluid to cool the material to be sterilized via the connection section.

Moreover; according to a fifth aspect of the present invention, there is provided a sterilization apparatus comprising:
a container for sterilization including a containing section in which a material to be sterilized is stored, and an opening via which a fluid is supplied/discharged to sterilize and cool the stored material to be sterilized;
a chamber in which the container for sterilization is stored to sterilize the material by steam having a high pressure and temperature in a sterilization position;
a moving mechanism which moves the container for sterilization between the sterilization position where the material to be sterilized is sterilized and a cooling position where the material to be sterilized is cooled; and
cooling means for supplying/recovering the cooling fluid which cools the material to be sterilized via the opening in the cooling position.

### Brief Description of the Drawings

FIGS. 1A to 1C are explanatory views of a procedure of sterilization by a sterilization apparatus according to a first embodiment of the present invention;
FIG. 2 is a block diagram showing a schematic constitution of a sterilization apparatus 100;
FIG. 3 is a flowchart showing the procedure of a high pressure steam sterilization treatment by the sterilization apparatus 100;
FIG. 4 is a time chart showing a state in which a pressure in a chamber 4 changes in accordance with progress of a series of sterilization treatment; and timings at which each corresponding element is ON, OFF, or opened/closed;
FIGS. 5A, 5B are explanatory views of a second embodiment of the present invention;
FIG. 6 is a diagram showing a constitution of a connection section for connecting a valve portion of a nozzle on the side of a sterilization cast 150 to a nozzle on the side of the unit;
FIGS. 7A, 7B are diagrams showing a sterilization apparatus 101 including a nozzle 170 for injecting a cooling gas into the sterilization cast 150 or a peel pack 153 after a sterilization step;
FIGS. 8A, 8B are diagrams showing the constitution of a conventional autoclave device;
FIGS. 9A, 9B are diagrams showing appearance of the autoclave device according to a third embodiment of the present invention;
FIG. 10 is a diagram showing that a sterilization tray lid of the autoclave device is opened;
FIG. 11 is a diagram showing details of a cooling structure for a material to be sterilized in the autoclave device;
FIG. 12 is an explanatory view showing an electric constitution of the third embodiment of the present invention;
FIG. 13 is a flowchart showing an operation of the third embodiment of the present invention;
FIG. 14 is a time chart showing the operation of the third embodiment of the present invention;
FIGS. 15A, 15B are diagrams showing the appearance of the autoclave device according to a fourth embodiment of the present invention;
FIGS. 16A, 16B are diagrams showing the constitution of a sterilization tray 412;
FIG. 17 is a diagram showing the constitution of the autoclave device according to a fourth embodiment of the present invention;
FIG. 18 is an explanatory view of an endoscope apparatus according to a fifth embodiment of the present invention;
FIG. 19 is an explanatory view of the constitution of a containing case and cold blast device according to the fifth embodiment of the present invention;
FIG. 20 is an explanatory view of another constitution of the containing case and cooling device according to a sixth embodiment of the present invention;
FIG. 21 is an explanatory view of another constitution of the containing case and cooling device according to a seventh embodiment of the present invention;
FIG. 22 is an explanatory view of a hanger by which an endoscope is held and a cap member in which a tip end of an insertion portion is disposed;
FIG. 23 is an explanatory view of an endoscope inspection trolley in which an endoscope containing chamber is disposed; and
FIG. 24 is an explanatory view of a bath in which the containing case is contained.

### Best Mode for Carrying Out the Invention

Each embodiment of the present invention will hereinafter be described in detail.

### (Outlines of First and Second Embodiments)

Outlines of first and second embodiments of the present invention will be described. In a first constitution, a door of a chamber automatically opens after a sterilization step ends, a material to be sterilized in the chamber is taken out of the chamber by a take-out device, and rapidly cooled by a cooling gas from a cooling nozzle attached to the take-out device, and the door of the chamber is closed. In accordance with this constitution, the material can rapidly be cooled without troubling an operator or without cooling the inside of the chamber. Therefore, load reduction of a sterilization operation and reduction of a time in a cycle can be realized.

Moreover, in a second constitution, the cooling gas passed through a germfree filter is sprayed onto the material contained in a sterilization cast or peel pack. In accordance with this constitution, the material can efficiently be cooled, there is an advantage that the material can immediately be reused, and efficiency of in-hospital services is promoted.

### (Details of First Embodiment)

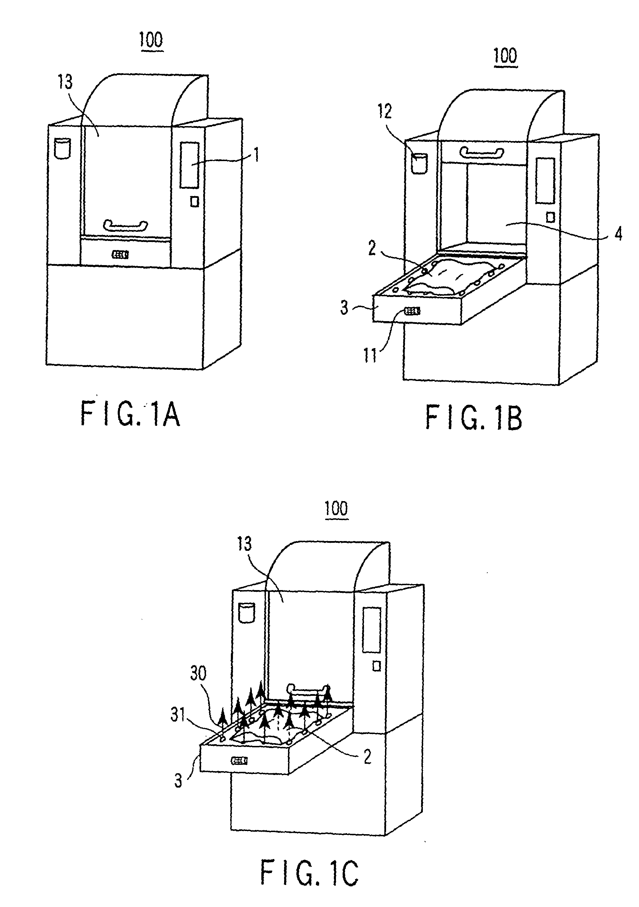

FIGS. 1A, 1B, 1C are explanatory views of a procedure of sterilization by a sterilization apparatus according to a first embodiment of the present invention.

FIG. 1A shows a condition in which the material to be sterilized is sterilized by a sterilization apparatus 100. In this case, a door 13 is closed. Reference numeral 1 denotes an operation panel. FIG. 1B shows a condition after the sterilization by the sterilization apparatus 100 ends. That is, when the sterilization ends, it is confirmed by a safety check sensor 11 that there is not any person or obstacle on a front surface of the sterilization apparatus 100. When the check can be performed, the door 13 is moved upwards and opened. Next, a drawer tray 3 on which a material to be sterilized 2 is laid is drawn. In this state, an inner part of a chamber 4 appears. Reference numeral 12 denotes an alarm lamp. FIG. 1C shows a condition in which the material 2 is cooled in preparation for the next sterilization. That is, the door 13 is closed again in order to keep heat in the chamber 4. Moreover, in order to promote the cooling of the materials 2, a cooling air 30 is spouted via cooling gas jet ports 31 disposed in the drawer tray 3. The cooling gas jet ports 31 and cooling air 30 constitute cooling means.

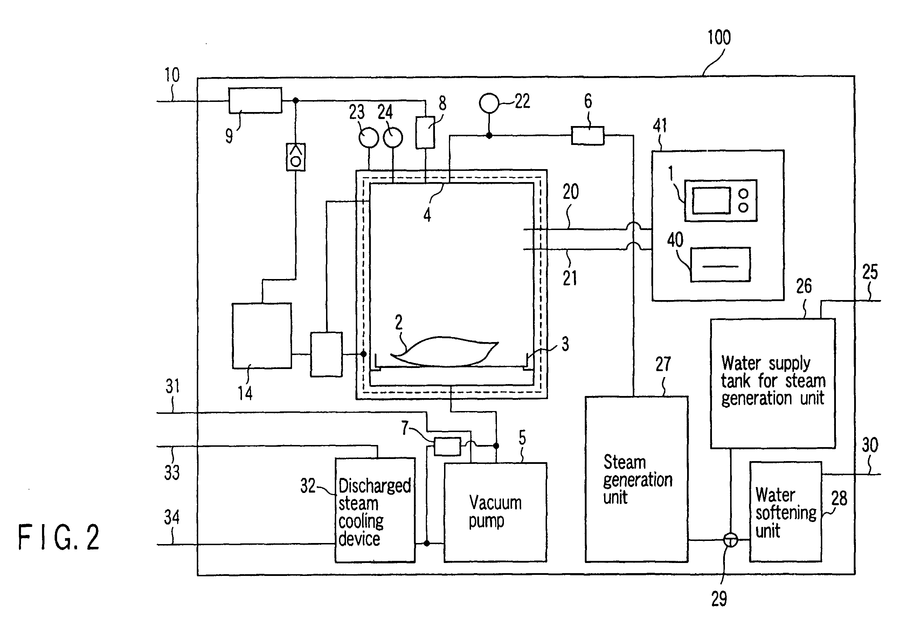

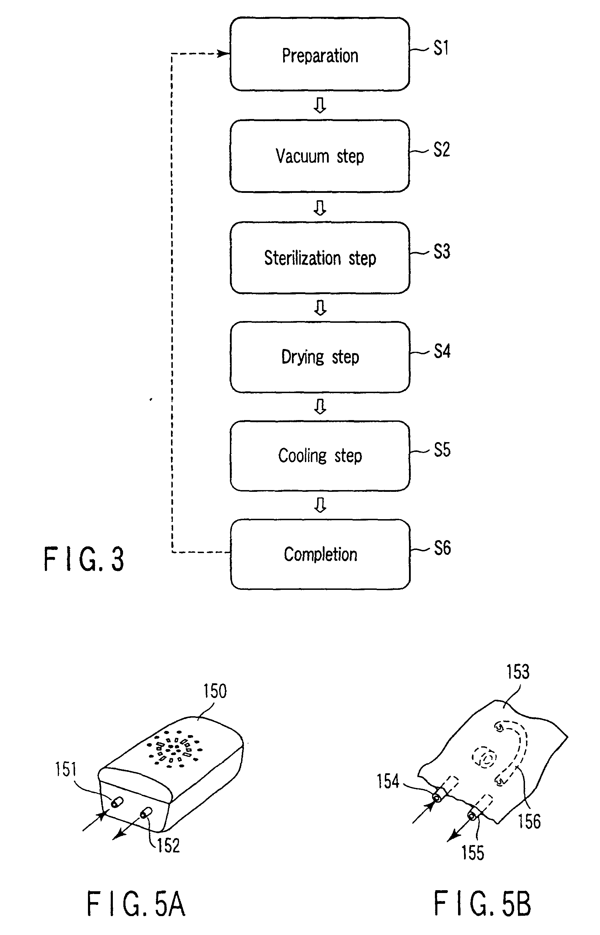

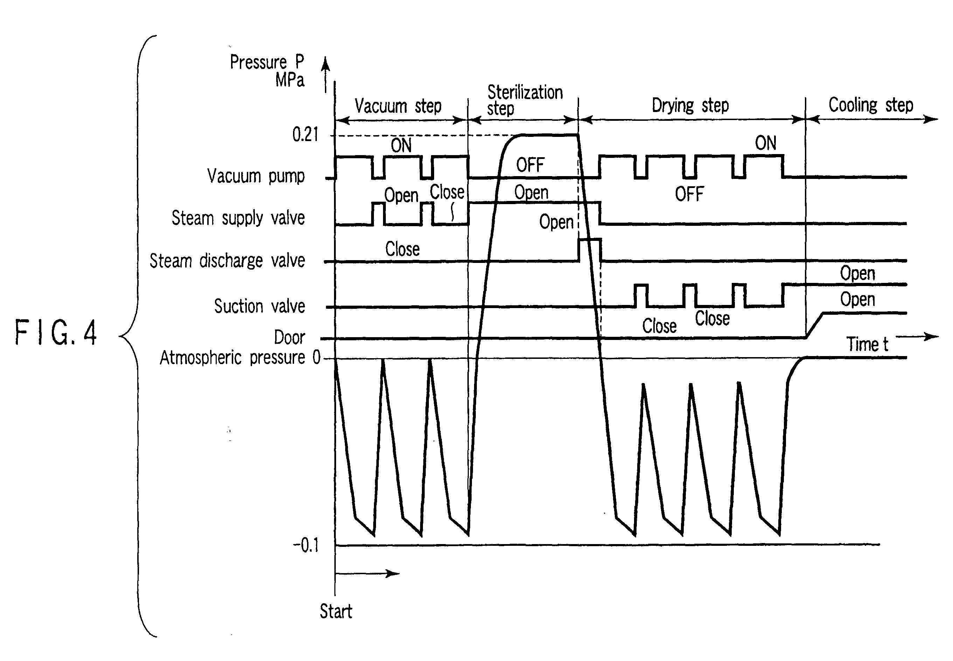

FIG. 2 is a block diagram showing a schematic constitution of a sterilization apparatus 100, and FIG. 3 is a flowchart showing the procedure of a high pressure steam sterilization (autoclave) treatment by the sterilization apparatus 100. The high pressure steam sterilization treatment will be described with reference to FIGS. 1A to 3.

First, in a preparation process (step S1), a user operates a switch of the operation panel 1, and opens the door 13 of the sterilization apparatus 100 to draw out the drawer tray 3. Next, the material 2 held in the sterilization pack is laid on the drawer tray 3, the switch of the operation panel 1 is operated to store the drawer tray 3 in the chamber 4, and the door 13 is closed.

After confirming that the door 13 has been closed, a sterilization treatment start switch of the operation panel 1 is pressed. The door 13 of the sterilization apparatus 100 is locked, and a series of sterilization treatment starts. For the series of sterilization treatment, first the pressure in the chamber 4 is reduced in a vacuum step (step S2) of drawing air in the chamber 4 with a vacuum pump 5. When the pressure in the chamber 4 drops to a preset pressure (e.g., -0.09 MPa), the vacuum pump 5 is stopped. Subsequently, a steam supply valve 6 is opened to introduce a high-pressure steam produced by a steam generation unit 27 into the chamber 4, and the flow enters a sterilization step (step S3). The steam is fed into the chamber 4 until the inside of the chamber reaches a preset sterilization temperature (e.g., at 135°C). To raise the efficiency, vacuum drawing and steam supply may alternately be repeated.

When the temperature inside the chamber 4 reaches the set sterilization temperature, the inside of the chamber 4 is kept at the temperature for a preset sterilization time (e.g., five minutes). When the sterilization time elapses, a steam discharge valve 7 for discharging the high-pressure steam in the chamber 4 to the outside of the chamber 4 is opened. The discharged high-pressure steam is cooled and changed into water by a cooling water supplied from a cooling water supply port 33 in a discharged steam cooling device 32, and is thereafter discharged via a drain discharge port 34.

After discharging the steam, the steam discharge valve 7 is closed to reduce the pressure in the chamber 4 again with the vacuum pump 5. This is a drying step (step S4), the inside of the chamber 4 is reduced in the pressure at a high-temperature state, and therefore droplets are rapidly vaporized to dry the inside.

When a preset drying time (e.g., ten minutes) elapses, the vacuum pump 5 stops, and a suction valve 8 is opened to return the inside of the chamber 4 to an atmospheric pressure. In order to prevent the inside of the chamber 4 from being contaminated by constantly existing germs in the air, in general, a filter which is called a germfree filter 9 and which has a filter mesh of 0.2 microns or less is attached to an outside air intake port 10 of the suction valve 8. When the inside of the chamber 4 returns to the atmospheric pressure, with the safety check sensor 11 disposed on the front surface of the sterilization apparatus 100, it is checked whether or not there is any person or obstacle on the front surface of the sterilization apparatus 100. While the alarm lamp 12 is lit, or an alarm sound is emitted, the door 13 automatically opens, and the drawer tray 3 on which the material 2 is laid is discharged to the outside of the chamber 4.

At this time, a height of the material 2 is detected by a sensor (not shown) which is disposed in the chamber 4 to detect a size of the material 2. Moreover, when the door 13 is opened up to a necessary minimum height in consideration of the detected height, heat insulation in the chamber 4 can be improved. After the drawer tray 3 is discharged, the door 13 is quickly closed for the heat insulation in the chamber 4.

Next, the flow enters a cooling step (step S5). The cooling gas which has been sent from a compressor 14 in the unit to cool the material 2 is spouted from the cooling gas jet port 31 disposed in the drawer tray 3. When a preset time elapses, the cooling gas is spouted, the alarm sound and alarm lamp 12 are turned off, and the cooling step ends.

At this time, by a temperature sensor disposed on the drawer tray 3, the temperature of the drawer tray 3 or the material 2 is measured, and a temperature (e.g., 40°C) at which the person touches the drawer tray 3 or the material 2 but does not suffer burns. Then, the spouted cooling gas is stopped, the alarm sound and alarm lamp 12 are turned off, and the cooling or warning does not have to be done for an extra time. Then, a series of sterilization treatment is completed (step S6).

A user moves the material to be sterilized 2 to a storage place or a place for use from the drawer tray 3. In a state in which this drawer tray 3 is discharged, the next material to be sterilized 2 is laid on the drawer tray 3, and the sterilization step can immediately be started. Therefore, an operation efficiency is satisfactory, and trouble and time are saved. Next, when not in use, the switch of the operation panel 1 may also be operated to store the drawer tray 3 in the chamber 4.

It is to be noted that in FIG. 2, reference numerals 21, 22, 23, 24 denote pressure gauges, and 20 denotes a temperature sensor. The operation panel 1 constitutes a control box 41 together with a recording device 40. A water supply tank 26 for the steam generation unit connected to a manual water supply port 25, and a water softening unit 28 connected to a water supply port 30 for the steam are connected to the steam generation unit 27 via a changeover valve 29.

A time chart of FIG. 4 shows a state in which the pressure in the chamber 4 changes in accordance with progress of the above-described series of sterilization treatment, and timings at which each corresponding element described above is ON, OFF, or opened/closed. It is to be noted that vacuum pump 5 is repeatedly turned ON, OFF in an ON period of the vacuum pump 5. The ON, OFF timing corresponds to a waveform of the atmospheric pressure.

### (Details of Second Embodiment)

A second embodiment of the present invention will hereinafter be described. FIG. 5A shows a sterilization cast 150, and FIG. 5B shows a peel pack 153. Each of these is used to store and sterilize the material by an autoclave device and to hold the subsequent sterile state. In the sterilization cast 150, an injection nozzle 151 and exhaust nozzle 152 are disposed, and the valves are attached to the respective nozzles 151, 152 so as to prevent germs in the outside air from entering the sterilization cast 150. The high-pressure steam does not easily penetrate the inside, unless an outside air pressure is high.

The valve of the injection nozzle 151 has such a mechanism as to be opened when a nozzle of an outside instrument for injecting the gas for the cooling is connected to the valve. The gas for the cooling can be injected from the outside. Instead of these valves, the germfree filter may be attached to the injection nozzle 151 to prevent contamination from the outside.

Similarly, an injection nozzle 154 and exhaust nozzle 155 are also disposed in the peel pack 153 shown in FIG. 5B, and the valves are disposed in the respective nozzles 154, 155. The functions of these valves are similar to those in the sterilization cast 150.

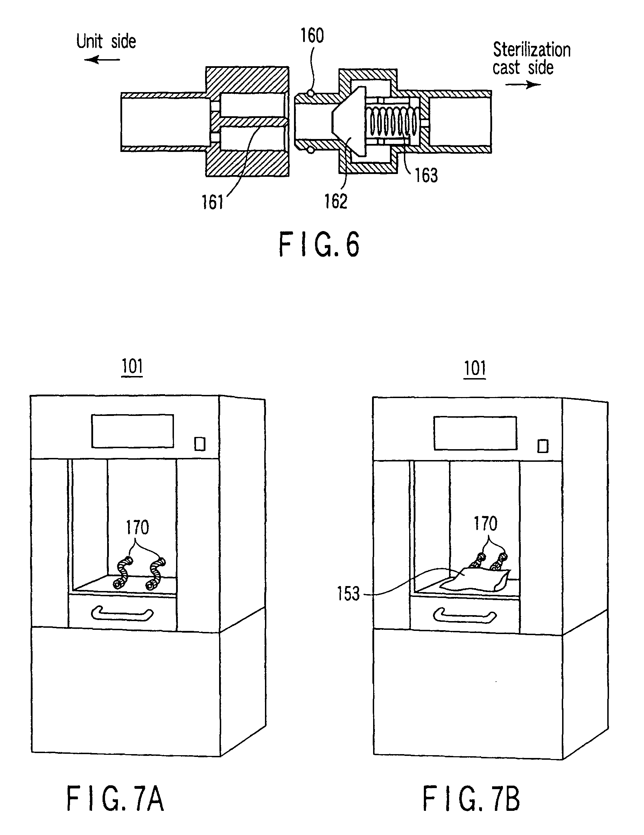

FIG. 6 shows a constitution of a connection section for connecting a valve portion of a nozzle on the side of the above-described sterilization cast 150 to a nozzle on the side of the unit. In the valve portion of the sterilization cast 150, a spring 163, a valve body 162, and an O ring 160 are disposed. A valve open pin 161 is disposed in the nozzle on the unit side. When the nozzle of the sterilization cast 150 is not connected to that on the unit side, the valve body 162 is urged by the spring 163 to close a channel, and therefore the gas does not flow. However, when the nozzle of the sterilization cast 150 is connected to that on the unit side, the valve open pin 161 pushes the valve body 162 to form a gas channel. Therefore, the cooling gas can be passed on the sterilization cast 150 side from the unit side.

FIGS. 7A and 7B show a sterilization apparatus 101 including nozzles 170 for injecting the cooling gas into the sterilization cast 150 or the peel pack 153 after the sterilization step. FIG. 7A shows a state before the material is set, and FIG. 7B shows a state in which a material to be sterilized 156 stored in the peel pack 153 shown in FIG. 5B is set.

The operator connects the injection nozzle 154 of the peel pack 153 to the jet nozzle 170 in the chamber 4, and sets the pack in the chamber 4. Thereafter, after the switch of the operation panel 1 is operated to close the door 13, the sterilization treatment is started. The drying step ends and the inside of the chamber 4 returns to the atmospheric pressure in the same manner as in the first embodiment. After the inside of the chamber 4 returns to the atmospheric pressure, the cooling gas passed through the germfree filter 9 attached to the sterilization apparatus 101 from the side of the unit 101 is fed into the peel pack 153 via the injection nozzle 154.

The fed gas is discharged to the outside of the unit 101 via the exhaust nozzle 155. When the material 156 is cooled, the door of the unit 101 opens, and the peel pack 153 can be taken out. In this case, since the cooling gas substantially passes only through the peel pack 153, and the chamber 4 itself is not cooled very much, any problem is caused in starting the next sterilization operation.

According to the above-described first and second embodiments, the following effects are produced. That is, when the material to be sterilized is an instrument that contacts a human body, and the instrument is to be used immediately after the high pressure steam sterilization, the instrument has heretofore been taken out of the sterilization apparatus immediately after the sterilization, and has to be cooled with a blower, and the like. For this, the operation has to continue until the sterilization ends, and this has been troublesome. However, in accordance with the above-described embodiment, a cooling promotion step is safely and automatically performed after the sterilization ends, and therefore this remarkably contributes to reduction of personnel expenses. Moreover, in a state in which the chamber is kept warm, the material can be cooled, the treatment of the next material to be sterilized can be started soon, and therefore the operation efficiency is also enhanced. Moreover, it can be confirmed from a remote area that the material has been cooled at a safe temperature, and this is therefore very convenient.

### (Outlines of Third and Fourth Embodiments)

Outlines of third and fourth embodiments of the present invention will be described. In a first constitution, a tray for sterilization (including ventilating holes) for storing materials to be sterilized, such as an endoscope, and holding a sterile state even after the sterilization, and air feed means (pump, compressor, duct, and the like) for feeding air to positively dry or cool the endoscope contained inside are disposed in the vicinity of the outside of the chamber of an AC device. The constitution is also characterized in that the fed air positively passes in or around the tray.

Moreover, in a second constitution, the air feed means (pump, compressor, and the like) is brought into common use with a pump for sealing a chamber lid of the autoclave device and a compressor for the drying.

Furthermore, a third constitution includes: opening/closing means for opening/closing the lid for the chamber of the autoclave device; slide means for guiding the material in the chamber to the outside of the chamber in conjunction with the operation of the opening/closing means; cooling means for cooling the material; and control means for executing a control to open the opening/closing means in response to the end of the sterilization treatment and to drive the slide means and the cooling means and to cool the material.

In the above-described constitution, when the materials to be sterilized such as the endoscope are contained in the tray for sterilization (including the ventilating holes), and thereafter disposed in the chamber of the autoclave device to perform the sterilization step, the high-pressure steam is supplied into the chamber, and the temperature of the endoscope main body rises, for example, to 135°C that is a sterilization temperature. Since the ventilating holes are disposed in the sterilization tray, the high-pressure steam is also supplied into the sterilization tray.

Thereafter, when the sterilization and drying steps end, the pressure in the chamber drops, and the chamber lid can be opened. When the chamber lid is opened, the slide means operates to guide the sterilization tray (material to be sterilized) in the chamber to the outside of the chamber, and the sterilization tray is moved in an operation position of the cooling means disposed in the vicinity of the chamber lid. The cooling means operates in conjunction with this operation to cool the sterilization tray. The fed air is highly efficiently fed to the ventilating holes of the sterilization tray and the outer surface by a duct disposed in the cooling means.

The materials to be sterilized (endoscope, and the like) are cooled in a short time by this series of operation, total required time of a conventional sterilization treatment step is reduced, and an efficient endoscope inspection can be carried out.

### (Details of Third Embodiment)

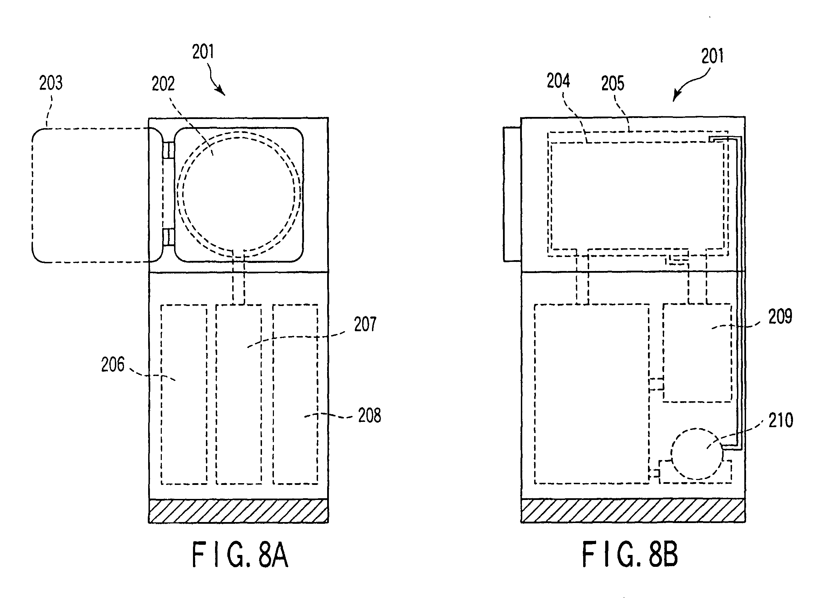

A third embodiment will hereinafter be described in detail with reference to the drawings. First, the constitution of a conventional autoclave device will be described with reference to FIGS. 8A, 8B. In general, an autoclave device main body 201 includes: a high-pressure container (hereinafter referred to as a chamber main body 202) for inserting (disposing) the material to be sterilized; and a lid (door) 203 for the chamber which keeps airtightness of the chamber main body 202 and which opens/closes in inserting/removing the material. For the chamber main body 202, a steam generation device 209 for producing the high-pressure steam in performing the high pressure steam sterilization (hereinafter referred to as the autoclave) is connected to a tank 206 for steam water supply, and is further connected to a chamber inner tube 204 and chamber outer tube 205 of the chamber main body 202 via a steam supply conduit.

Moreover, the chamber inner tube 204 is connected to a vacuum pump 210 for once discharging the air and evacuating the inside of the chamber to replace the air with the high-pressure steam and to perform a pretreatment with a satisfactory thermal efficiency before the autoclave and for promoting the drying of the material after the end of the autoclave via an exhaust conduit.

Since the vacuum pump for use in the autoclave device discharges the steam in general, a water-sealing pump is used, and a tank 208 for the vacuum pump, for supplying water necessary for the operation of the pump, is disposed in the autoclave device. Furthermore, when the sterilization step ends in the steps of the autoclave, the steam inside the chamber is discharged to the outside of the chamber via a steam discharge conduit, but this steam has a very high temperature, and has to be cooled to a certain degree. For this, a tank 207 for cooling the steam is mounted in the autoclave device. Additionally, many components such as a control substrate for controlling each unit, various electromagnetic valves, a temperature sensor, and a safety valve are used, but the description is omitted here.

Next, the operation of the above-described conventional autoclave device will be described. First, the material is stored in the chamber main body 202, and the lid for the chamber 203 is securely closed. Usually, in order to securely seal lid for the chamber 203 and the chamber main body 202, an air packing driven by the compressor is disposed in a contact portion between the lid for the chamber 203 and the chamber main body 202, and steam leakage at the high pressure is prevented. Next, when a sterilization start switch (not shown) is turned on, the vacuum pump 210 operates to discharge the air from the chamber inner tube 204. When extra air remains, the high-pressure steam securely prevents a sterilization defect from being caused in the chamber by presence of a portion (cold spot) which does not contact of the material.

When the inside of the chamber main body 201 has vacuum (about -0.1 MPa), the high-pressure is next supplied to the chamber inner tube 204 from the steam generation device 209. When the steam is appropriately supplied, the temperature of the chamber inside and the material reaches a sterilization condition, for example, at 135°C, a sterilization timer (not shown) operates, and the sterilization step is performed, for example, at 135°C for five minutes. When the sterilization step ends for five minutes, next the steam of the chamber inner tube 204 is discharged into the tank 207 for cooling the steam via the steam discharge conduit, and further the vacuum pump 210 again operates in order to dry the material. The steam is supplied to the chamber outer tube 205 in a standby state of the unit.

These drying steps end, the material to be sterilized is naturally cooled, and all the steps are completed. The material is taken out of the chamber, left to stand, and naturally cooled in some case. In a conventional method, after these sterilizing and drying steps, the materials are naturally cooled, and therefore a waiting time of 30 minutes or more is required until the materials are next used.

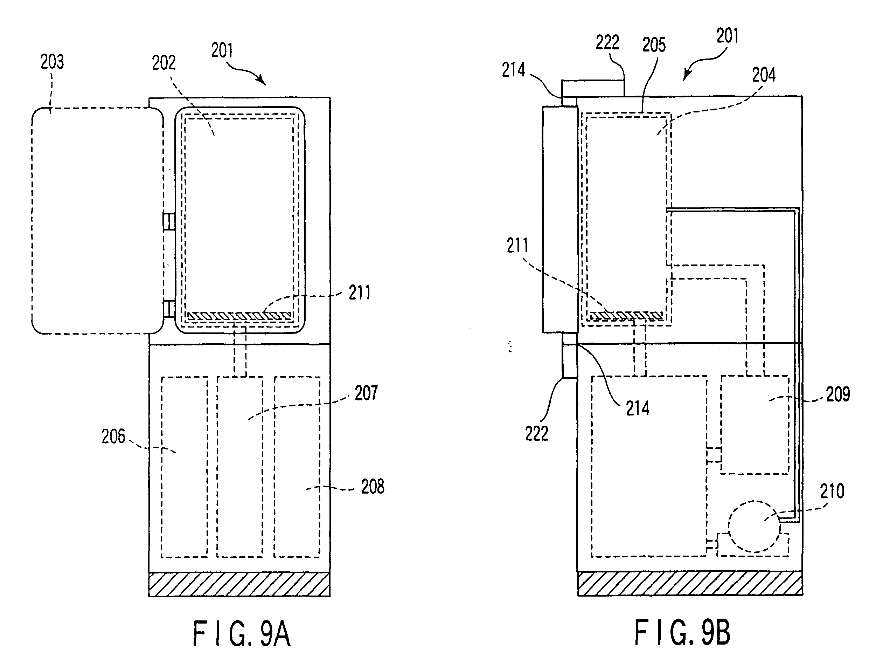

Next, the details of the third embodiment of the present invention will be described. FIGS. 9A, 9B are diagrams showing the appearance of the autoclave device according to the third embodiment. The third embodiment is characterized in that a chamber portion of the autoclave device (FIGS. 8A, 8B) described in the related art is changed to a chamber having a shape to be disposed in a vertical direction (hanging) in order to improve usability in accordance with the shape of the endoscope that is the material.

The chamber main body 202 is connected to the lid for the chamber 203 which keeps the airtightness of the chamber and which is opened/closed in inserting/removing the material, and the steam supply conduits for supplying the high-pressure steam in performing the autoclave, and is further connected to the steam discharge conduit for discharging the steam from the chamber after the end of the autoclave. The rest of the constitution is similar to that of the conventional unit.

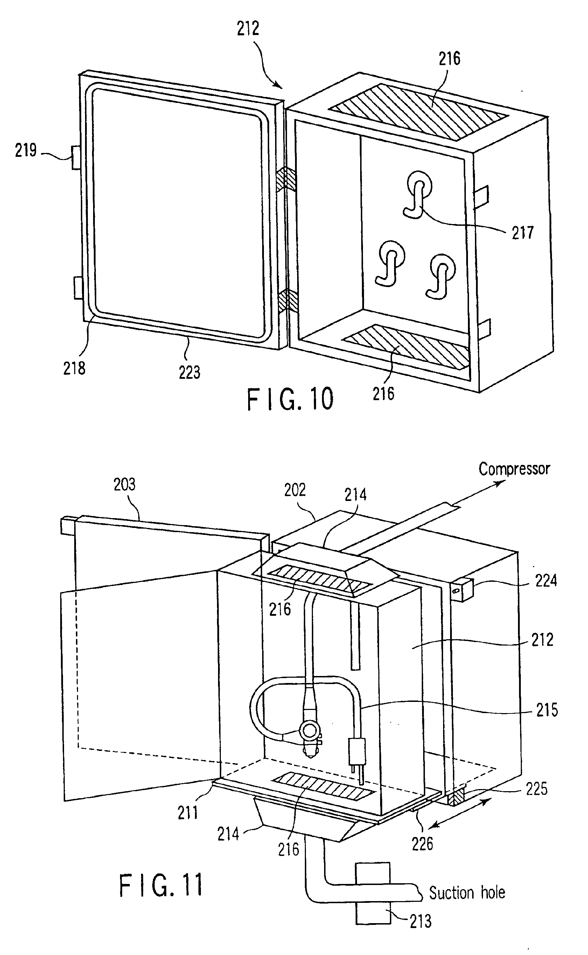

In the third embodiment, further, in the chamber, as shown in FIG. 10, a sterilization tray 212 of such a type as to dispose the endoscope in the vertical direction (hanging) in accordance with the shape of endoscope that is the material to be sterilized is stored, and further, a slide member 211 for moving the sterilization tray 212 to the outside of the chamber is disposed.

This slide member 211 is constituted, for example, of a slide rail and a motor for driving. Moreover, for the sterilization tray 212, as shown in FIG. 10, hooks 217 for suspending the endoscope which is the material are disposed. The hooks 217 are disposed in positions where the disposing of the endoscope is regulated so as to avoid a curved state of an endoscope insertion portion at the time of the autoclave, and are disposed, for example, in three positions in the third embodiment.

Moreover, since the high-pressure steam enters upper/lower surfaces of the sterilization tray (shaded portion of FIG. 10), filters 216 including ventilating holes (additionally, the filter passes steam and air) constituted so as to prevent other impurities (germs, dust, and the like) from entering the tray are disposed. Moreover, in the sterilization tray 212, a seal member 218 and clamp members 219 are disposed for closely attaching a tray main body to a sterilization tray lid 223 in order to securely hold the sterile state after the sterilization.

Next, a cooling structure of the material will be described. The details of the cooling structure are shown in FIG. 11. Two upper and lower air ducts 214 are disposed in upper and lower portions of an opening in the chamber main body 202 in order to circulate the air in the sterilization tray 212 and on the outer periphery of the tray. In the present embodiment, the upper air duct 214 is constituted to suck the air, and the lower air duct 214 has a function of air exhaust. The upper air duct 214 is connected to a suction port of the compressor, and the lower air duct 214 is connected to a suction hole via an air filter 213 which prevents impurities from coming in.

Moreover, the sterilization tray 212 is structured to be detachably attached to the slide member 211 disposed in the chamber, for example, via magnets (not shown). When the sterilization treatment is performed, the tray is fixed to the slide member 211. In conjunction with the opening/closing operation of the chamber lid 203, the tray moves into the chamber at the time of the sterilization, and moves to the outside of the chamber after the drying step. A limit SW 224, which is opening/closing detection means for detecting the opening/closing operation, is disposed in the vicinity of the chamber lid 203.

In the sterilization tray 212, the upper and lower filters 216 are disposed to pass the high-pressure steam and to prevent other impurities (germs, dust, and the like) from entering the tray. When the sterilization tray 212 moves out of the chamber by the operation of the slide member 211, the positions of the air ducts 214 disposed in the upper and lower portions of the opening of the chamber main body 202 to suck and discharge the air, and the filters 216 disposed in the upper and lower portions of the sterilization tray 212 are controlled so as to be fixed in positions where the air can efficiency be sent out.

Moreover, the number of the air ducts 214 disposed in the upper and lower portions of the opening in the chamber main body 202 can be selected from one to a plurality of ducts (in parallel) in accordance with an effect of cooling, and the most efficient number may be selected. The ventilating hole of the sterilization tray 212 may also serve as the lower air duct 214.

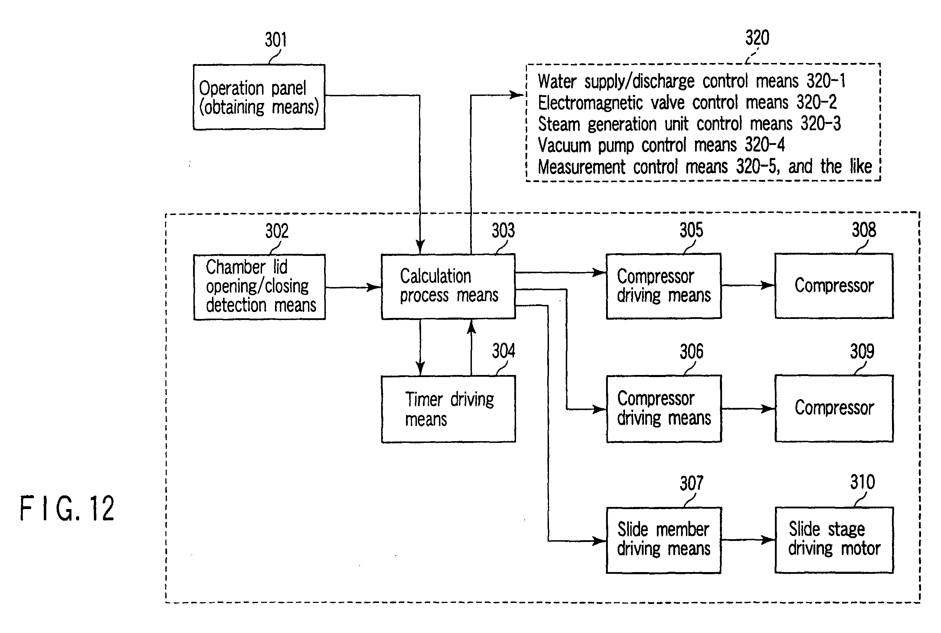

Next, an electric constitution of the third embodiment of the present invention will be described with reference to FIG. 12. The present autoclave device is constituted of: an operation panel (input means) 301 via which operation conditions of the device and start and stop of each step are inputted; water supply/discharge control means 320-1 which controls the water supply to the device and water discharge from the device based on these input signals; electromagnetic valve control means 320-2 for controlling the opening/closing of various pipes; steam generation unit control means 320-3 for producing the steam to be supplied to the chamber; vacuum pump control means 320-4 for setting the inside of the chamber to be vacuum and for promoting the drying of the material after the completion of the sterilization step; measurement control means 320-5 for measuring operation situations of the device and values of temperature, pressure and the like in the chamber; and calculation process means 303 for generally controlling various control means 320 described above.

Furthermore, in addition to the basic constitution, provided are: chamber lid opening/closing detection means 302 for detecting the opening/closing of the lid for the chamber 203, compressor driving means 305 for controlling the operation of a compressor 308 which operates during a usual sterilization step, compressor driving means 306 for controlling the operation of a compressor 309 which sucks the air for cooling the material in conjunction with an opening/closing signal of the chamber lid 203, slide member driving means 307 for controlling a slide stage driving motor 225 for conveying the sterilization tray 212 to the outside from the inside of the chamber in conjunction with the opening/closing signal of the chamber lid 203, and timer driving means 304 for counting an operation time of the compressor driving means 306 are connected to the calculation process means 303. Here, when running conditions of the device and conduit constitution are devised, the compressors 308 and 309 are not constituted independently, and can be united for use.

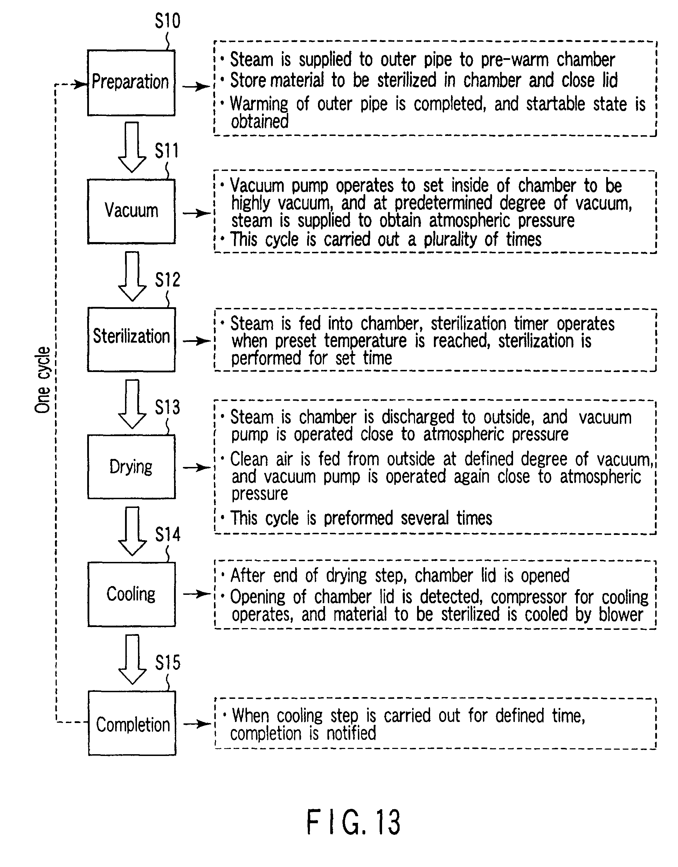

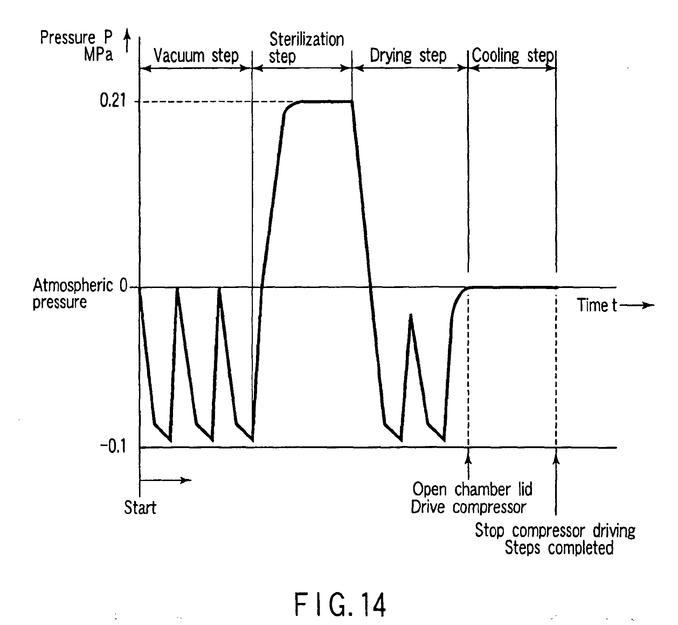

Next, the operation of the third embodiment will be described with reference to FIGS. 13, 14. One cycle of the sterilization step of the third embodiment is as follows. As a "preparation" step (step S10), the steam is supplied to a chamber outer can beforehand. Next, for example, the endoscope which is the material to be sterilized is stored in the sterilization tray 212, and attached to the chamber main body 202, thereby completing the preparation. Next, when the sterilization step is started, the flow enters a "vacuum" step (step S11), the vacuum pump 210 operates, and the air inside the chamber main body 202 is discharged.

As shown in FIG. 14, when the inside of the chamber main body 202 is a vacuum (about -0.1 MPa), the high-pressure steam is supplied to the inside of the chamber main body 202 from the steam generation device 209 until the atmospheric pressure is obtained.

This step is repeated until the remaining air in the chamber is replaced with the high-pressure steam. FIG. 14 shows that the steps are carried out three times. When the "vacuum" step (step S11) ends, next a "sterilization" step is carried out (step S12), and the high-pressure steam is fed into the chamber. When the temperature of the inside of the chamber and the endoscope in the sterilization tray 212 reaches, for example, the sterilization condition of 135°C, the sterilization timer operates, and the sterilization step is operated, for example, at 135°C for five minutes. At this time, the sterilization conditions are appropriately set for reasons such as heat resistance of the material.

When a time set with the sterilization timer elapses, the steam in the chamber is discharged into the tank 207 for cooling the steam. Close to the atmospheric pressure, the vacuum pump 210 operates again to shift to a "drying" step (step S13). At a defined degree of vacuum, outside clean air is fed into the chamber. This step is repeated several times in accordance with the type of the material to be sterilized. FIG. 14 shows that the step is carried out twice. Next, the "drying" step (step S13) ends, and the lid for the chamber 203 is opened to shift to a "cooling" step (step S14). The slide stage driving motor 225, which is slide member driving means, operates in conjunction with the opening/closing signal of the limit SW 224 disposed in the vicinity of the lid for the chamber 203, and the sterilization tray 212 is conveyed to the outside from the inside of the chamber.

At this time, the filters 216 of the sterilization tray 212 are controlled to be fixed in positions opposite to the air ducts 214 disposed in the upper and lower portions of the opening of the chamber main body 202 disposed in the unit main body to feed and discharge the air by the calculation process means 303. Furthermore, in conjunction with the operation, the compressor 202 which sucks the air for cooling the material to be sterilized is driven to supply the air for the cooling via the filters 216 of the sterilization tray 212, and the material is automatically forcibly cooled to complete the "cooling" step (step S14) and to complete one cycle of the sterilization treatment. At this time, the sterilization tray 212 is also cooled. This cooling is completed in about five to ten minutes.

According to the above-described third embodiment, the sterilization operation is facilitated. Moreover, the material can automatically be cooled after the end of the sterilization step (step S12) and the drying step (step S13). Therefore, the total sterilization treatment time can largely be reduced, and the sterilization can efficiently be carried out between cases.

### (Details of Fourth Embodiment)

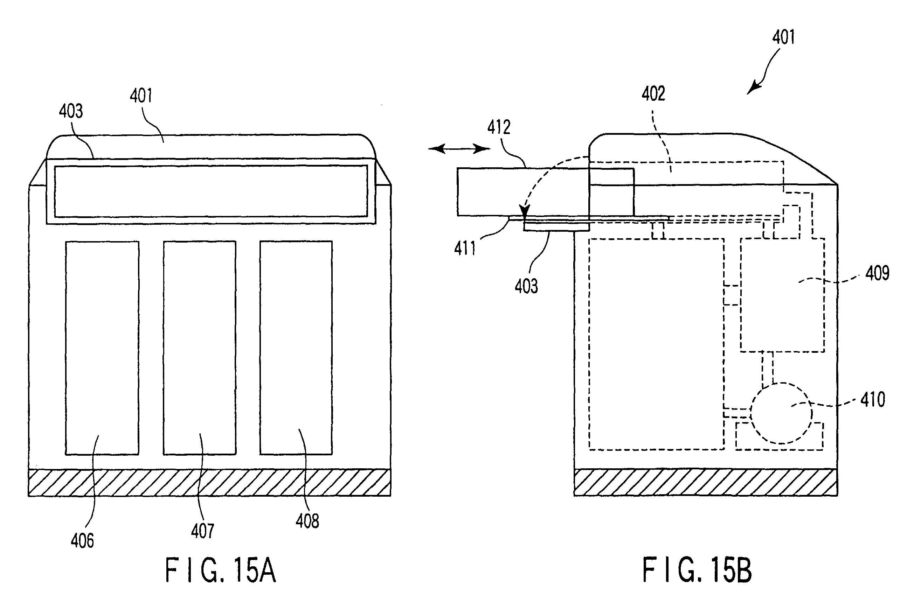

A fourth embodiment of the present invention will hereinafter be described in detail with reference to the drawings. FIGS. 15A, 15B are diagrams showing the appearance of the autoclave device according to the fourth embodiment of the present invention. The fourth embodiment is characterized in that a chamber portion is applied to a chamber having a horizontal plane shape in order to further facilitate the using of the autoclave device described with reference to FIGS. 9A, 9B.

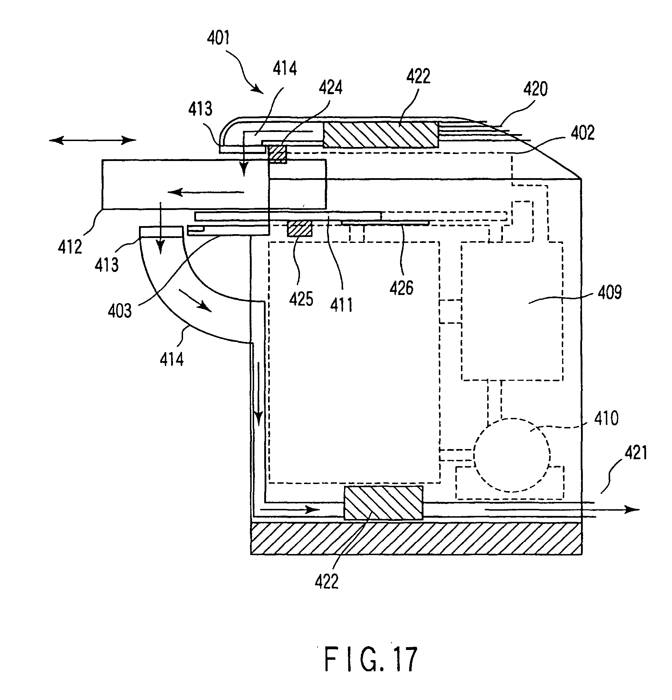

For a chamber main body 402, a lid for the chamber 403 which keeps the airtightness of the chamber and which is opened/closed in inserting/removing the material, and a slide member 411 for moving the sterilization tray 412 to the outside of the chamber are disposed. The slide member 411 is coupled, for example, to a slide rail 426 and motor for driving 425 (see FIG. 17).

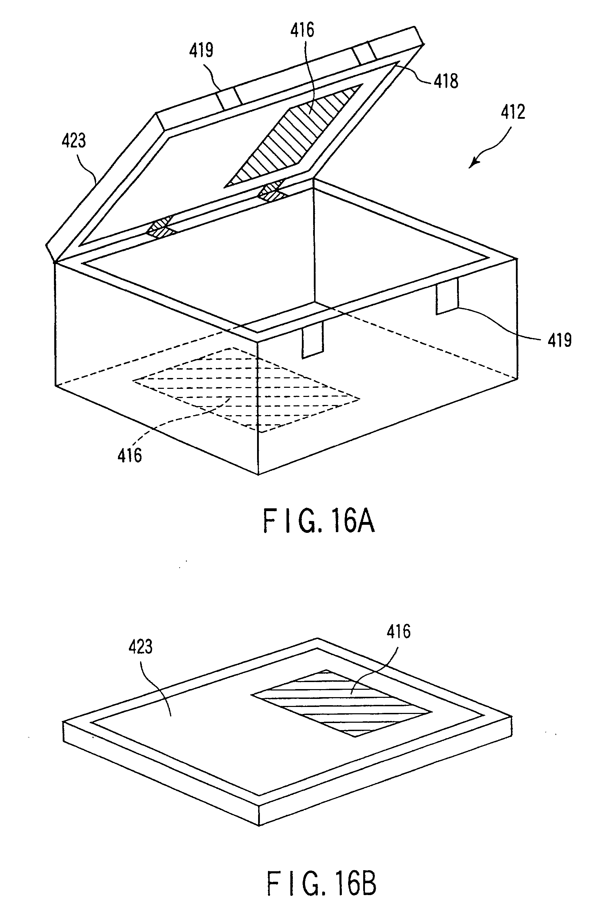

Moreover, as shown in FIGS. 16A, 16B, the sterilization tray 412 is constituted to be disposed in a horizontal form. In order to securely hold the sterile state after the sterilization, a seal member 418 and clamp members 419 are disposed to closely attach the tray main body to a sterilization tray lid 423. Moreover, as shown by the slashed parts of FIGS. 16A, 16B, filters 416 including the ventilating holes so that the high-pressure steam enters and the other impurities (germs, dust, and the like) are prevented from entering the tray (additionally, this filter 416 passes the steam and air) are disposed on the lid of the tray and on the upper/lower surface of the main body.

As shown in FIG. 16A, the sterilization tray lid 423 may include either a united structure with the tray main body as shown in FIG. 16A or a separate structure as shown in FIG. 16B.

Since a basic constitution for performing the autoclave is similar to that of the third embodiment, the description is omitted. The cooling structure in the fourth embodiment will hereinafter be described. FIG. 17 shows the details of the cooling structure. Two upper and lower air ducts 414 are disposed in the upper and lower portions of the opening in the chamber main body 402 in order to feed (discharge) the air and exhaust. In the fourth embodiment, the upper air duct 414 is constituted to feed the air, and the lower air duct 414 has the function of the air exhaust. For the upper air duct 414, a suction hole 420 is disposed in the suction port of the first compressor (upper part 422 of FIG. 17), an air filter 413 is disposed in a discharge port, the air filter 413 is disposed in the suction port of the lower air duct 414, and the air duct is connected to the second compressor (lower part 422 of FIG. 17). An air exhaust hole of the second compressor is disposed in a rear surface of the device.

Moreover, the structure is detachably attached to the slide member 411 disposed in the chamber via a magnet, in the same manner in the third embodiment. In conjunction with the opening/closing operation of the chamber lid 403, the sterilization tray 412 moves into the chamber at the time of the sterilization, and moves to the outside of the chamber after the end of the drying step. Further, in the same manner as in the third embodiment, a limit SW 424, which is the opening/closing detection means for detecting the opening/closing operation, is disposed in the vicinity of the chamber lid 403.

In the sterilization tray 412, the filters are disposed in the upper/lower part to pass the high-pressure steam but to prevent the other impurities (germs, dust, and the like) from entering the tray. When the sterilization tray 412 moves out of the chamber, the operation of the slide member 411 is controlled by the slide rail 426 and the motor for the driving 425 to fix the positions of the air ducts 214 disposed in the upper/lower portions of the opening of the chamber main body 402 to feed (discharge) the air and to exhaust, and those of the filters disposed in the upper/lower portions of the sterilization tray 412 onto the positions where the air can efficiency be sent out.

In the fourth embodiment, two compressors are disposed to strongly cool the material, but depending on a degree of effect, the effect can be obtained without using the lower air duct or the second compressor.

Since the electrical constitution of the fourth embodiment of the present invention is substantially the same as that of the third embodiment described with reference to FIG. 12, the description herein is omitted. In the fourth embodiment, since two compressors for the cooling are used, compressor driving means for a third compressor separately from the compressor 309 is added (not shown).

Next, the operation of the fourth embodiment will be described. In the same manner as in the third embodiment, for example, the endoscope is stored in the sterilization tray 412, and attached to the chamber main body 402 of the autoclave device to start the sterilization step. Then, the vacuum pump 410 operates, and the air inside the chamber main body 402 is discharged. When the inside of the chamber main body 402 is a vacuum (about -0.1 MPa), next, the high-pressure steam is supplied to the inside of the chamber main body 402 from the steam generation device 409. When the steam is appropriately supplied, and the temperature of the inside of the chamber and the endoscope in the sterilization tray 412 reaches, for example, the sterilization condition of 135°C, the sterilization timer operates, and the sterilization step is carried out, for example, at 135°C for five minutes. Next, when the sterilization step ends, the steam in the chamber is discharged into the tank 207 for cooling the steam via the steam discharge conduit, and the vacuum pump 410 operates again to shift to the drying step.

When these drying steps end, and the lid for the chamber 403 is opened, the slide stage driving motor 425 operates in conjunction with the opening/closing signal of the lid for the chamber 403 by the slide member driving means, and the sterilization tray 412 is conveyed to the outside from the inside of the chamber.

At this time, the ventilating holes 416 of the sterilization tray 412 are controlled to be fixed in the positions opposite to the air ducts 414 disposed in the upper/lower portions of the opening of the chamber main body 402 disposed in the device main body to feed (discharge) the air and to exhaust. Further, in conjunction with the operation, the compressor 422 which sucks the air for cooling the material is driven to suck the air for the cooling via the ventilating holes 416 of the sterilization tray 412. When the material to be sterilized is automatically forcibly cooled, all the steps are completed. At this time, the sterilization tray 412 is also cooled (the cooling is completed in about five to ten minutes).

According to the above-described fourth embodiment, since the shape and disposing method of the chamber are improved, the sterilization operation is facilitated. Moreover, since the material can automatically be cooled after the end of the sterilizing and drying steps, the total sterilization treatment time can largely be reduced, and the sterilization can efficiently be carried out between the cases.

The above-described third and fourth embodiments produce the following effects.
1. Immediately after the end of the sterilizing and drying steps, the material to be sterilized can automatically be cooled.
2. Immediately after the end of the sterilizing and drying steps, the sterilization tray can automatically be cooled.
3. The endoscope can easily be disposed in the chamber in the autoclave device (fourth embodiment).
4. Since the material and the sterilization tray are forcibly cooled, the material and the sterilization tray can be used in a short time after the end of the sterilization, and the endoscope inspection can be carried out with good efficiency.

### (Fifth Embodiment)

An embodiment of the present invention will hereinafter be described with reference to the drawings.

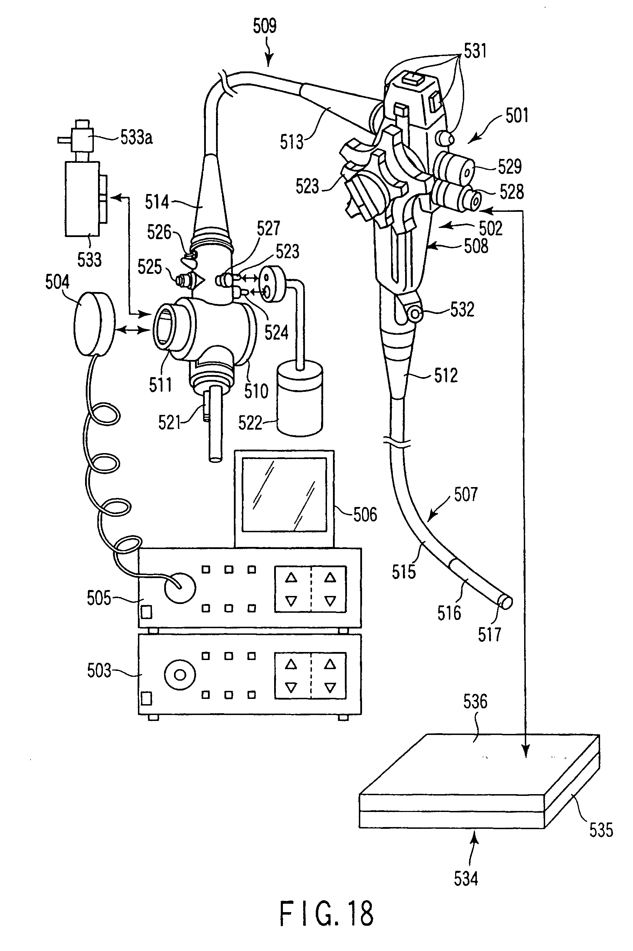

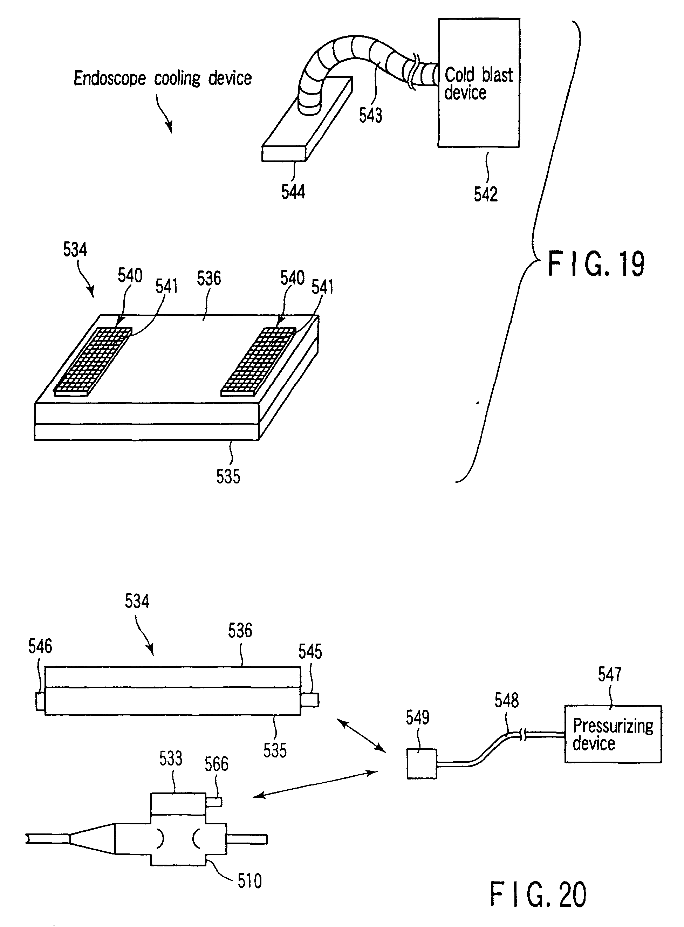

FIGS. 18 and 19 show a fifth embodiment of the present invention, FIG. 18 is an explanatory view of an endoscope apparatus, and FIG. 19 is an explanatory view of the constitution of a containing case and cold blast device.

As shown in FIG. 18, an endoscope apparatus 501 of the present embodiment is mainly constituted of:
an endoscope 502 including image pickup means (not shown); a light source device 503 which is detachably connected to the endoscope 502 to supply an illuminative light to a light guide (not shown) disposed in the endoscope 502; a video processor 505 which is connected to the endoscope 502 via a signal cable 504 to control the image pickup means of the endoscope 502 and which processes an image signal obtained by the image pickup means into a video signal; and a monitor 506 which displays the video signal outputted from the video processor 505.

The endoscope 502 is constituted in such a manner that the endoscope is cleaned after used in observation and treatment and can thereafter be sterilized in the high pressure steam sterilization.

The endoscope 502 is constituted of: an elongated insertion portion 507 having flexibility; an operation portion 508 disposed on a base end of the insertion portion 507; a connection cord 509 extending from a side portion of the operation portion 508 and having flexibility; and a connector portion 510 disposed in the end of the connection cord 509 and detachably connected to the light source device 503. On the side portion of the connector portion 510, an electric connector portion 511 detachably connectable to the signal cable 504 connected to the video processor 505 is disposed.

In this electric connector portion 511, a ventilating portion (not shown) connecting the inside to the outside of the endoscope 502 is disposed. Therefore, in the high pressure steam sterilization, the electric connector portion 511 is detachably connected to a waterproof cap 533 including a pressure adjustment valve. Moreover, a pressure adjustment valve 533a is disposed in this waterproof cap 533.

In a connection portion between the insertion portion 507 and operation portion 508, an insertion portion side breaking stop member 512 including an elastic member is disposed in order to prevent a sharp bend in the connection portion. Moreover, an operation portion side breaking stop member 513 is similarly disposed in the connection portion between the operation portion 508 and connection cord 509, and a connector portion side breaking stop member 514 is also similarly disposed in the connection portion between the connection cord 509 and connector portion 510.

In order from an operation portion 508 side, the insertion portion 507 is constituted of: a flexible tubular portion 515 having flexibility; a bendable portion 516 bendable by the operation of a bend operation knob 530 positioned in the tip end of this flexible tubular portion 515 and disposed in the operation portion 508; and a tip end 517 disposed on a tip-end side of the bendable portion 516 and including an optical observation system and optical illumination system in a connected manner.

The tip end 517 includes: a gas/water feed nozzle which spouts cleaning solution and gas toward an optical member (not shown) on the outer surface of the optical observation system by a gas or water feed operation of a gas/water feed operation button 528 disposed in the operation portion 508; and a suction port which is a tip-end opening of a treatment instrument channel (not shown) to pass the treatment instrument disposed in the insertion portion 507 or to suck liquid in a body cavity. Moreover, a solution feed port is also disposed which is opened toward an observation object to spout solution.

In the connector portion 510, a gas supply cap 521 detachably connected to a gas supply source (not shown) built in the light source device 503, and a water feed tank pressurizing cap 523 and solution supply cap 524 detachably connected to a water feed tank 522 which is a solution supply source are disposed. Moreover, a suction cap 525 connected to a suction source (not shown) to perform suction via the suction port is disposed. Furthermore, an injection cap 526 connected to water feed means (not shown) for feeding water via the solution feed port is disposed. An earth terminal cap 527 is disposed to return a leak current to a high-frequency treatment device (not shown), when a high-frequency treatment, and the like are performed, and the high-frequency leak current is produced in the endoscope 502.

In the operation portion 508, in addition to the gas/water feed operation button 528 for performing the gas/water feed operation and the bend operation knob 530 for performing the bend operation of the bendable portion, a suction operation button 529 for performing a suction operation, a plurality of remote switches 531 for remotely operating the video processor 505, and a treatment instrument insertion port 532 which is a base-end opening communicating with the treatment instrument channel are disposed.

When the endoscope 502 constituted as described above is subjected to high pressure steam sterilization after the use, the endoscope 502 is stored in a containing case for sterilization 534 (hereinafter abbreviated as the containing case) which also serves as a storage container of an endoscope cooling device. The containing case 534 is constituted of a tray 535 and lid member 536. In the tray 535 and lid member 536, a plurality of ventilating holes (not shown) capable of passing water vapor are disposed, so that the water vapor can pass through the ventilating holes.

It is to be noted that a regulating portion (not shown) is disposed in the tray 535 of the containing case 534 in accordance with the shape of the endoscope 502. The regulating portion is formed in such a manner that the respective parts of the endoscope 502 are disposed in a predetermined position, and the insertion portion 507 having the flexibility is stored in an insertion portion regulating portion (not shown).

Here, the high pressure steam sterilization will be described.

In American standards ANSI/AAMI ST37-1992 approved by American Standards Association and issued by Medical Equipment Research and Development Association, which are typical conditions of the high pressure steam sterilization, the sterilization step in a pre-vacuum type is set to be performed at 132°C for four minutes, and the sterilization step in a gravity type is set to be performed at 132°C for ten minutes.

Moreover, temperature conditions at the time of the sterilization step of the high pressure steam sterilization differ with a type of a high pressure steam sterilization apparatus or a time of the sterilization step, but in general, a temperature range is set at about 115°C to 138°C. For some of the sterilization apparatuses, the temperature can be set at about 142°C.

Furthermore, time conditions differ with the temperature conditions of the sterilization step, but are set to about three to 60 minutes in general. Some other types of sterilization apparatuses can also be set to about 100 minutes.

In this step, the pressure in a sterilization chamber is generally set to about +0.2 MPa with respect to the atmospheric pressure.

The high pressure steam sterilization steps of the general pre-vacuum type includes a pre-vacuum step of bringing the inside of the sterilization chamber in which a sterilization object equipment is stored into a reduced pressure state before the sterilization step, and a sterilization step of thereafter feeding a high-pressure/temperature steam into the sterilization chamber to perform the sterilization.

The pre-vacuum step is a step for allowing the steam to permeate detailed portions of the sterilization object apparatus at the time of the subsequent sterilization step. When the pressure in the sterilization chamber is reduced, the high-pressure/temperature steam permeates the whole sterilization object apparatus. The pressure in the sterilization chamber in the pre-vacuum step is in general set to about -0.07 MPa to -0.09 MPa with respect to the atmospheric pressure.

After the sterilization step for drying the sterilization object apparatus after the sterilization, the drying step of bringing the inside of the sterilization chamber into the reduced pressure state again is sometimes included. In this drying step, the pressure in the sterilization chamber is reduced and the steam is removed from the sterilization chamber to promote the drying of the sterilization object apparatus in the sterilization chamber. The pressure in the sterilization chamber in this step is in general set to about -0.07 MPa to -0.09 MPa with respect to the atmospheric pressure.

To subject the endoscope 2 to the high pressure steam sterilization, the sterilization is performed in a state in which the waterproof cap 533 including the pressure adjustment valve 533a is attached to the electric connector portion 511. In this state, the pressure adjustment valve 533a of the waterproof cap 533 is in a closed state, the ventilating ports are closed by the waterproof cap 533, and the inside and outside of the endoscope 502 are brought in a watertight sealed state.

In the sterilization method including the pre-vacuum step, the pressure in the sterilization chamber drops in the pre-vacuum step, a pressure difference is generated such that the pressure of the outside of the endoscope 502 is lower than that of the inside, then the pressure adjustment valve 533a opens, and the inside of the endoscope 502 communicates with the outside via the ventilating ports. That is, a large pressure difference is prevented from being generated between the inside of the endoscope 502 and the inside of the sterilization chamber, and accordingly the endoscope 502 does not break by the pressure difference between the inside and outside of the endoscope 502.

The inside of the sterilization chamber is pressurized, and the pressure difference is generated in such a manner that the pressure inside the endoscope 502 is higher than that of the outside. Then, the pressure adjustment valve 533a closes. Accordingly, the high-pressure/temperature steam does not positively enter the endoscope 502 via the waterproof cap 533 and the ventilating ports. However, the high-temperature/pressure steam gradually enters the inside through O rings formed of a fluorine rubber or silicon rubber, which is sealing means disposed in an outer shell of the flexible tubular portion 515 formed of a polymeric material and a connection portion of an outer member of the endoscope 502.

Therefore, in the outer member of the endoscope 502, a pressure is generated which is obtained by addition of a pressure reduced in the pre-vacuum step and that added in the sterilization step and which is directed inwards from the outside.

It is to be noted that in the method including the pressure reduction step after the sterilization step, in the pressure reduction step, the pressure of the sterilization chamber decreases, accordingly the pressure difference is generated such that the pressure of the outside of the endoscope 502 is lower than that of the inside, and the pressure adjustment valve 533a substantially simultaneously opens. Accordingly, the inside of the endoscope 502 communicates with the outside via the ventilating ports to prevent a large pressure difference from being generated between the inside of the endoscope 502 and that of the sterilization chamber. Accordingly, the endoscope 502 does not break by the pressure difference between the inside and the outside.

Subsequently, the pressure reduction step ends, the inside of the sterilization chamber is pressurized, the pressure difference is caused such that the pressure outside the endoscope 502 is higher than that inside the endoscope, and the pressure adjustment valve 533a then closes.

When all the steps of the high pressure steam sterilization end, the pressure directed inwards from the outside is generated in the outer member of the endoscope 502 by the pressure reduction in the pressure reduction step.

Here, when the waterproof cap 533 is removed from the electric connector portion 511, the inside of the endoscope 502 communicates with the outside via the ventilating ports, the inside of the endoscope 502 also obtains the atmospheric pressure, and a load generated in the outer member of the endoscope 502 by the pressure difference is eliminated.

As shown in FIG. 19, for example, in the vicinity of opposite ends of the lid member 536 of the containing case 534 in a longitudinal direction, ventilator windows 540 including germ trapping filters 541 constituting the inlet ports or exhaust ports are disposed, respectively. When the tray 535 is covered with the lid member 536, the sealed state is obtained in an airtight manner excluding the ventilator windows 540 in which the germ trapping filters 541 are disposed.

The ventilator windows 540 are disposed in positions where the air is convected with respect to at least the insertion portion 507 of the endoscope 502 stored/disposed in the containing case 534.

Moreover, the ventilator window 540 can be connected to a connection portion 544 disposed in the tip end of a flexible hose 543 extended from a cold blast device 542 which is a gas driving device of an endoscope cooling device to feed the cold blast for cooling the endoscope 502 substantially in an airtight manner.

It is to be noted that also for the positions where the ventilator windows 540 are to be disposed, various variations are further considered. Moreover, when the endoscope including a soft insertion portion is stored in a predetermined state in the containing case 534, two ventilator windows 540 serving as the intake and discharge ports are preferably disposed in the positions where the cold blast flows with respect to at least the insertion portion 507.

Here, the cooling step of the endoscope 502 will be described.

There is a possibility that the temperature of the endoscope 502 stored in the containing case 534 immediately after the autoclave sterilization is at 40°C or more. To insert the endoscope into a patient's body for the inspections, it is waited until the temperature of the endoscope 502 is at 40°C or less, and a re-process time lengthens.

To solve the problem, in the present embodiment, immediately after the autoclave sterilization, one ventilator window 540 disposed in the lid member 536 constituting the containing case 534 is connected to the connection portion 544 of the cold blast device 542, and the cold blast device 542 is driven to feed the cold blast into the containing case 534.

Then, two ventilator windows 540 are positioned/disposed so that the air flows into the insertion portion 507. Therefore, at least the insertion portion 507 is soon cooled, and the endoscope 502 stored in the containing case 534 can quickly reach a temperature at which the endoscope can be inserted in the patient's body, that is, the inspection can be carried out.

At this time, since the cold blast from the cold blast device 542 is fed into the containing case 534 via the germ trapping filter 541, germs are prevented from entering the containing case 534 and the sterile state of the endoscope 502 is held.

In this manner, immediately after the autoclave sterilization, the cold blast from the cold blast device is fed into the containing case via the ventilator window disposed in the lid member of the containing case in which the endoscope is stored. Accordingly, immediately after the sterilization, the endoscope at the high temperature can quickly be cooled while the sterile state is held.

### (Sixth Embodiment)

FIG. 20 is an explanatory view of another constitution of the containing case and cooling device according to a sixth embodiment of the present invention. As shown, in the present embodiment, a suction cap 545 and discharge cap 546 are disposed on the opposite portions on a longitudinal side of the tray 535 constituting the containing case 534. In the caps 545, 546, although not shown, the germ trapping filters are disposed. Moreover, in the same manner as in the fifth embodiment, except the caps 545, 546, the tray 535 and lid member 536 are in the airtight sealed state.

The suction cap 545 is connectable to a connection cap 549 disposed in the tip end of a flexible tube 548 extending from a pressurizing device 547 for detecting leak water. The connection cap 549 is connected to the pressure adjustment valve 533a disposed in the waterproof cap 533 attached to the connector portion 510.

That is, the pressurizing device 547 of the present embodiment also serves as the cold blast device which feeds a gas via the connection cap 549 to cool the endoscope or feeds a cooling gas via the cooling mechanism to cool the endoscope in the same manner as in the fifth embodiment.

Therefore, according to the present embodiment, after the endoscope inspection, the connection cap 549 is attached to the pressure adjustment valve 533a of the waterproof cap 533 attached to the connector portion 510 of the endoscope 502 before the cleaning, and accordingly the inside of the endoscope 502 is pressurized by the pressurizing device 547 to detect the leak water. At this time, when a hole is made somewhere in the endoscope 502 and the airtightness is impaired, the pressurizing device 547 detects that.

On the other hand, immediately after the endoscope 502 is cleaned and the autoclave sterilization is performed, the connection cap 549 of the pressurizing device 547 is now connected to the suction cap 545 of the tray 535 to feed the gas into the containing case 534. Accordingly, in the same manner as in the fifth embodiment, the endoscope 502 in the containing case 534 is quickly cooled.

In this manner, in the present embodiment, the pressurizing device for detecting the leak water is used to feed the gas into the containing case and the endoscope can be cooled. Accordingly, a user can inexpensively constitute the endoscope cooling device to quickly cool the endoscope at the high temperature with the existing pressurizing device without newly purchasing the cooling device.

It is to be noted that, in general, the containing case 534 is formed of a hard resin or metal, but may also be constituted of a sterile cloth, and the suction cap 545, and the like may be attached to the sterile cloth. Moreover, the suction cap 545 is branched in the containing case 534, and the gas is applied to the outer surface of the endoscope 502. Additionally, the cap may also be constituted to be connected to the conduit of the endoscope 502 so that the gas is fed into the endoscope conduit to cool the endoscope.

### (Seventh Embodiment)

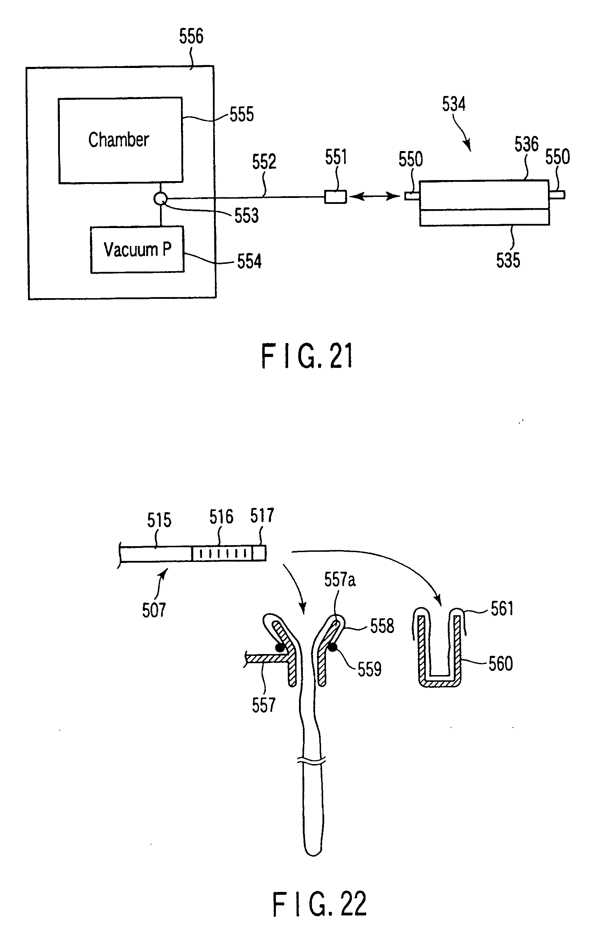

FIG. 21 is an explanatory view of another constitution of the containing case and cooling device according to a seventh embodiment of the present invention.

As shown, in the present embodiment, ventilating caps 550 are disposed on the opposite portions on the longitudinal side of the lid member 536 of the containing case 534. In the ventilating caps 550, in the same manner as in the embodiment described above, the germ trapping filters (not shown) are attached.

The ventilating cap 550 is connected to a connection cap 551 disposed in one end of a conduit 552 extending from an autoclave device 556. The other end of the conduit 552 is connected to a changeover valve 553, and this changeover valve 553 is disposed in the conduit to connect a chamber 555 in which the material to be sterilized is stored to a vacuum pump 554.

Therefore, immediately after the autoclave sterilization of the containing case 534 in which the endoscope 502 is stored, the connection cap 551 disposed in the conduit 552 is connected to the ventilating caps 550 of the containing case 534, the vacuum pump 554 is driven, and the changeover valve 553 is switched on a conduit 552 side to suck the air, so that the gas in the containing case 534 is convected to cool the endoscope 502.

On the other hand, the changeover valve 553 is switched on a chamber 555 side during the autoclave sterilization. Accordingly, the vacuum pump 554 functions with respect to the chamber 555, and a pre-vacuum stroke or a vacuum drawing/drying stroke is performed.

In this manner, the vacuum pump disposed in the chamber is used in the autoclave sterilization and the cooling of the endoscope, and accordingly the endoscope in the containing case can more quickly be cooled immediately after the sterilization. Accordingly, in the same manner as in the sixth embodiment, the user can inexpensively constitute the endoscope cooling device without newly purchasing the cooling device.

It is to be noted that temperature warning means may also be disposed in the containing case 534. Accordingly, the user can judge the temperature of the containing case 534 by the temperature warning means. Moreover, the temperature warning means may also be reversible seals to announce that the temperature is not more than or not less than a certain specific temperature.

Additionally, in the case for sterilizing the endoscope in the above-described Jpn. Pat. Appln. KOKAI Publication No. 5-337081, contamination prevention in a stored state after performing the autoclave has been described, but contamination prevention at the time of inspection start has not been described.

That is, a function of supplying energies of electricity, light, fluid, and the like to the endoscope 502, which is a function surely required at the time of the endoscope inspection, is OFF. In this case, there is a possibility that an operator is brought into a state in which a control switch disposed in a light source device or video processor has to be operated. In the conventional endoscope, a preventive measure has not been described with respect to contamination by the operation of the video processor or light source device which is connected to the endoscope and which has not been sterilized at the time of the use of the sterilized endoscope in the inspection.

Therefore, there has been a demand for an endoscope system in which the function disposed in the video processor or the light source device is performed without contaminating the endoscope after the sterilization and the endoscope inspection is performed while the sterilization is maintained.

To solve the problem, in the present embodiment, the function controlled by the switch disposed in the video processor 505 or the light source device 503 can also be controlled with the switch disposed in the endoscope 502. Accordingly, without contacting the non-sterilized video processor 505 or light source device 503, the necessary function is operated to prevent the contamination of the endoscope 502 after the sterilization.

Therefore, when the corresponding switch of the remote switches 531 disposed in the operation portion 508 of the endoscope 502 shown in FIG. 18 is operated, ON/OFF of a power supply of the video processor 505 or the light source device 503, ON/OFF of a gas supply pump disposed in the light source device 503, or an output level of a light amount of the light source device 503 can be controlled. It is to be noted that these functions are basically necessary for the endoscope 502, and are not usually operated during the inspection, and the control switch is disposed in the light source device 3 or the video processor 505.

That is, the present embodiment is constituted in such a manner that various switches corresponding to various controls are disposed in the light source device 503 or the video processor 505, and the light source device 503 or the video processor 505 can be controlled by the remote switches 531 of the endoscope 502.

Accordingly, when the remote switches 531 of the sterilized endoscope 502 are operated, for example, the function of supplying the energies of the electricity, light, fluid and the like to the endoscope 502 is controlled to start the inspection without contacting the non-sterilized light source device 503 or video processor 505 by the operator.

It is to be noted that for the remote switches 531, by one operation of one specific switch, the output function of all the energies necessary for the above-described inspection for the endoscope 502 may also be controlled.

Moreover, an operation instruction of the remote switch 531, and functions achievable by the instruction may also be displayed on the video processor 505 or monitor 506.

Furthermore, initial setting (adjustment) of a white balance of the optical observation system essential before the inspection may also be performed based on an instruction signal outputted from this remote switch 531.

In this manner, various functions of the video processor, light source device, and the like are performed with the remote switches disposed in the operation section of the endoscope. Accordingly, the functions essential for the inspection, disposed in the light source device or video processor, can be controlled without contaminating the sterilized endoscope.

Moreover, during standby before the start of the inspection, the insertion portion 507 of the endoscope 502 needs to be prevented from being contaminated. Therefore; for example, a disposable cover 558 is disposed on a hanger 557 disposed in the vicinity of an endoscope inspection trolley (not shown) on which the light source device 503 and video processor 505 are mounted and including a hold portion 557a having a conical shape as shown in FIG. 22, and the sterilized insertion portion 507 is hooked on the hanger 557 on which the disposable cover 558 is disposed. Accordingly, the sterilized insertion portion 507 is prevented from directly contacting the hanger 557 or another non-sterilized peripheral material before the start of the inspection.

It is to be noted that elastic members 559 such as a rubber ring are disposed on one end of the disposable cover 558, and this end may be fixed to a constricted portion of the hold portion 557a. Therefore, the disposable cover 558 can be changed to a new one every inspection.

Moreover, a cap member 560 on which the tip end 517 of the insertion portion 507 is to be disposed and which is formed of a white resin is also covered with a cap cover 561. Accordingly, the tip end 517 of the insertion portion 507 can be disposed in the cap member 560 before the inspection to take the white balance.

Also in this case, since the non-sterilized cap member 560 is covered with the cap cover 561, the tip end 517 is prevented from directly contacting the non-sterilized cap member 560.

This cap member 560 may also be formed of white paper which is an inexpensive sterile material so as to be disposable. Moreover, for the disposable cover 558 or the cap cover 561, a grasp portion to be grasped during the mounting on the hanger 557 or the cap member 560 is disposed in a portion other than a portion contacting the endoscope 502. Accordingly, the portion which contacts the endoscope 502 is prevented from being contaminated during the mounting.

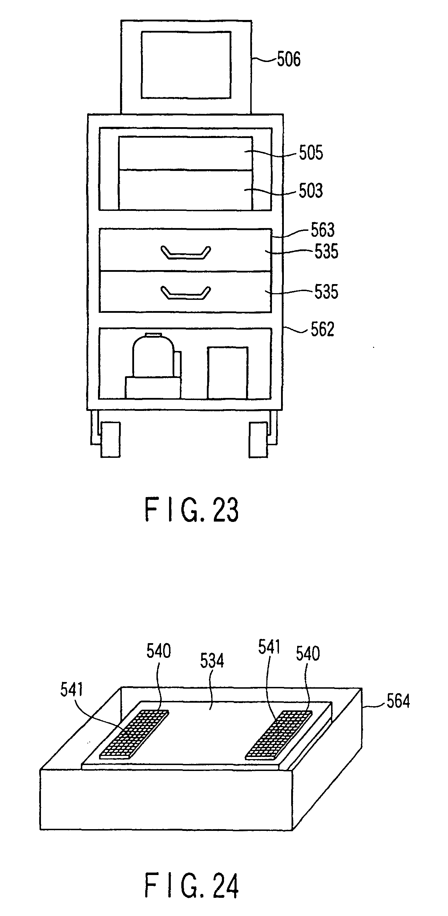

Furthermore, during the standby before the start of the inspection, in order to prevent the insertion portion 507 of the endoscope 502 from being contaminated, as shown in FIG. 23, an endoscope storage chamber 563 may be disposed in an endoscope inspection trolley 562. For this endoscope storage chamber 563, the trays 535 to store the endoscope 502 in a predetermined state are constituted like drawers.

In this manner, when the endoscope storage chamber 563 is disposed in the endoscope inspection trolley 562, as compared with the holding of the endoscope in a hanger mode before the inspection, the contamination of the endoscope after the sterilization by the contacting of the peripheral apparatus by the endoscope can securely be prevented.

Moreover, since the endoscope storage chamber 563 is capable of storing the containing case in a state in which the tray is covered with the lid member, the contamination of the endoscope can more securely be prevented.

It is to be noted that a temperature sensor is disposed in the vicinity of the CCD (not shown) built in the tip end 517 of the insertion portion 507, and a signal line of the temperature sensor is extended in the vicinity of the electric connector portion 511. Accordingly, the electric connector portion 511 is connected to a separate detection apparatus, the temperature around the CCD is measured, and it may be notified to the user whether or not the CCD can be prevented from being broken at the temperature. Accordingly, it can be notified to the user whether or not the breakage of the CCD can be prevented even with the connection of the signal cable 504 to the electric connector portion 511.

Moreover, as shown in FIG. 24, a bath 564 may also be disposed to store the containing case 534 described in the fifth embodiment. Cold solutions such as water are pooled in this bath 564. It is to be noted that a depth of the bath 564 is set so as to prevent the germ trapping filters 541 from being submerged under water in a full state of the solution.

In this manner, when the bath capable of storing the containing case is prepared, and the containing case is put in the bath and immersed in the solution immediately after the autoclave sterilization, the containing case can quickly be cooled.

It is to be noted that the present invention is not limited to the above-described embodiments, and can variously be modified without departing from the scope of the present invention.

### Industrial Applicability

According to the present invention, there can be provided a sterilization apparatus in which a material to be sterilized can rapidly be cooled without any operator's trouble or without cooling the inside of a chamber so as to realize reduction of a load of a sterilization operation and reduction of a time in a cycle.

Moreover, according to the present invention, it is possible to provide a sterilization apparatus in which the material is efficiently cooled and is immediately reusable so as to promote efficiency of in-hospital services.

Furthermore, it is possible to provide a sterilization apparatus in which a cooling time of an endoscope after high pressure steam sterilization is reduced and user's usability is enhanced and efficient endoscope instrument can be performed.

Additionally, it is possible to provide a container for sterilization in which the endoscope is quickly cooled without being contaminated immediately after autoclave sterilization.

## Claims

1. A sterilization apparatus comprising:
a chamber in which a material to be sterilized is stored to sterilize the material by steam having a high temperature and pressure; and
a cooling mechanism which cools the material in a state isolated thermally from the inside of the chamber.

2. The sterilization apparatus according to claim 1, further comprising:
an opening/closing mechanism which opens/closes the chamber; and
a control section which opens the opening/closing mechanism with the end of the sterilization step and which drives the cooling mechanism to cool the material to be sterilized.

3. The sterilization apparatus according to claim 1, further comprising:
an opening/closing mechanism which opens/closes the chamber; and
moving means for moving the material to be sterilized stored in the chamber to the outside of the chamber,
wherein the cooling mechanism cools the material moved to the outside of the chamber by the moving means, and
the sterilization apparatus further comprises a control section which opens the chamber after the end of the sterilization step and closes the chamber and keep warm the inside of the chamber after moving the material in the chamber to the outside of the chamber by the moving means and cools the material by the cooling mechanism.

4. A container for sterilization comprising:
a containing section in which a material to be sterilized is stored;
an intake port and an exhaust port of a gas, at least either one of which is connectable to a gas circulation mechanism to circulate the gas inside the containing section; and
a germ trapping filter disposed in at least the intake port.

5. The container for sterilization according to claim 4, wherein the container can be autoclaved.

6. The container for sterilization according to claim 4, wherein the gas circulation mechanism is a vacuum pump disposed in an autoclave device.

7. The container for sterilization according to claim 4, wherein the gas circulation mechanism is a pressurizing device which is detachably attached to the material to be sterilized and which changes a pressure inside the material.

8. A container for sterilization comprising:
a containing section in which a material to be sterilized is stored;
a connection section via which a fluid to sterilize and cool the stored material is supplied/discharged with respect to the outside; and
a blocking mechanism which is disposed in the connection section and which can communicate to supply/discharge the fluid in a connected state of the connection section and which blocks off the supply/discharge of the fluid in a non-connected state of the connection section.

9. The container for sterilization according to claim 8, wherein a germ trapping filter is further disposed in the connection section.

10. A sterilization apparatus, comprising:
a container for sterilization including a containing section in which a material to be sterilized is stored, a connection section which supplies/discharges a fluid to sterilize/cool the stored material with respect to the outside, and a blocking mechanism which is disposed in the connection section and which can communicate to supply/discharge the fluid in a connected state of the connection section and which blocks off the supply/discharge of the fluid in a non-connected state of the connection section;
a chamber in which the material or the container for sterilization is stored to sterilize the material by steam having a high pressure and temperature; and
fluid supply means capable of supplying at least one of a sterilization fluid to sterilize the material and a cooling fluid to cool the material via the connection section.

11. The sterilization apparatus according to claim 10, wherein the container for sterilization includes a sterilization case and a sterilization pack, and the sterilization case and the sterilization pack include a nozzle which is the connection section and which supplies/discharges a fluid to sterilize and cool the stored material to be sterilized with respect to the outside.

12. A sterilization apparatus comprising:
a container for sterilization including a containing section in which a material to be sterilized is stored, and an opening via which a fluid is supplied/discharged to sterilize and cool the stored material;
a chamber in which the container for sterilization is stored to sterilize the material by steam having a high pressure and temperature in a sterilization position;
a moving mechanism which moves the container for sterilization between the sterilization position where the material is sterilized and a cooling position where the material is cooled; and
cooling means for supplying/recovering the cooling fluid which cools the material in the cooling position via the opening.

13. The sterilization apparatus according to claim 12, further comprising:
opening/closing means for opening/closing the chamber; and
control means for opening the opening/closing means with end of the sterilization treatment, moving the material to be sterilized to the cooling position by the moving mechanism and cooling the material by the cooling means.

## Amended claims

### Amended claims under Art. 19.1 PCT

**1.** (amended). A sterilization apparatus comprising:
a chamber in which a material to be sterilized is stored and a sterilizing step is performed to sterilize the material with steam having of high temperature and high pressure;
an opening/closing mechanism which opens and closes the chamber; and
a cooling mechanism which cools the material sterilized in the chamber, in a state isolated thermally from the inside of the chamber; and
a control section which drives the cooling mechanism as the opening/closing mechanism opens the chamber after the sterilizing step ends, thereby to cool the material.

**2.** (amended). The sterilization apparatus according to claim 1, wherein:
the cooling mechanism has moving means for moving the material out of the chamber, and cooling means for cooling the material moved out of the chamber by the moving means; and
the control section has operation-processing means for driving the moving means as the opening/closing means opens the chamber after the sterilizing step ends, thereby to move the material out of the chamber, and then driving the cooling means to cool the material.

**3.** (Deleted)

**4.** A container for sterilization comprising:
a containing section in which a material to be sterilized is stored;
an intake port and an exhaust port of a gas, at least either one of which is connectable to a gas circulation mechanism to circulate the gas inside the containing section; and
a germ trapping filter disposed at least in the intake port.

**5.** The container for sterilization according to claim 4, wherein the container can be autoclaved.

**6.** The container for sterilization according to claim 4, wherein the gas circulation mechanism is a vacuum pump disposed in an autoclave device.

**7.** The container for sterilization according to claim 4, wherein the gas circulation mechanism is a pressurizing device which is detachably attached to the material to be sterilized and which changes a pressure inside the material.

**8.** (amended). A container for sterilization comprising:
a containing section in which a material to be sterilized is stored;
a connection section connected to means for supplying and discharging fluid that cools or sterilizes the material, provided in the containing section and configured to supply and discharge the fluid to and from the containing section; and
a blocking mechanism provided in the connection section and configured to supply and discharge the fluid while the connection section remains connected to the means for supplying and discharging the fluid, and to block off a passage for supplying and discharging the fluid while the connection section remains disconnected from the means for supplying and discharging the fluid.

**9.** The container for sterilization according to claim 8, wherein a germ trapping filter is further disposed in the connection section.

**10.** (amended). A sterilization apparatus, comprising:
a container for sterilization including a containing section in which a material to be sterilized is stored, a connection section which supplies/discharges a fluid to sterilize/cool the stored material with respect to the outside, and a blocking mechanism which is disposed in the connection section and which can communicate to supply/discharge the fluid in a connected state of the connection section and which blocks off the supply/discharge of the fluid in a non-connected state of the connection section;
a chamber in which the material or the container for sterilization is stored to sterilize the material by steam having a high pressure and temperature; and
fluid supply means capable of supplying at least one of a sterilization fluid to sterilize the material and a cooling fluid to cool the material via the connection section.

**11.** The sterilization apparatus according to claim 10, wherein the container for sterilization includes a sterilization case and a sterilization pack, and the sterilization case and the sterilization pack include a nozzle which is the connection section and which supplies/discharges a fluid to sterilize and cool the stored material to be sterilized with respect to the outside.

**12.** A sterilization apparatus comprising:
a container for sterilization including a containing section in which a material to be sterilized is stored, and an opening via which a fluid is supplied/discharged to sterilize and cool the stored material;
a chamber in which the container for sterilization is stored to sterilize the material by steam having a high pressure and temperature in a sterilization position;
a moving mechanism which moves the container for sterilization between the sterilization position where the material is sterilized and a cooling position where the material is cooled; and
cooling means for supplying/recovering the cooling fluid which cools the material in the cooling position via the opening.

**13.** The sterilization apparatus according to claim 12, further comprising:
opening/closing means for opening/closing the chamber; and
control means for opening the opening/closing means with end of the sterilization treatment, moving the material to be sterilized to the cooling position by the moving mechanism and cooling the material by the cooling means.
